# EUROPEAN PATENT SPECIFICATION

(11) **EP 2 498 909 B1**
(45) Date of publication and mention of the grant of the patent: **27.08.2014**
(21) Application number: 10829032.1
(22) Date of filing: 03.11.2010
(51) Int. Cl.: B01J 31/22, C07C 1/213, C07C 11/00, C07F 15/00, C07C 67/333, C07C 6/04, C11C 3/14

(54) **METATHESIS CATALYST AND PROCESS FOR USE THEREOF**
METATHESEKATALYSATOR UND VERFAHREN ZU SINER VERWENDUNG
CATALYSEUR DE MÉTATHÈSE ET SON PROCÉDÉ D'UTILISATION

(30) Priority: 09.11.2009 US 259514 P
(43) Date of publication of application: 19.09.2012
(73) Proprietor: ExxonMobil Chemical Patents Inc., Baytown, TX 77520 (US)
(72) Inventor: HAGADORN, John, R., Houston, TX 77035 (US); HOLTCAMP, Matthew, W., Huffman, TX 77336 (US); BEDOYA, Matthew, S., Humble, TX 77346 (US)
(74) Representative: Mareschal, Anne
(86) International application number: PCT/US2010/055309
(87) International publication number: WO 2011/056881

(56) References cited:
- WO-A2-2008/046106
- US-A1- 2005 070 750
- US-A1- 2007 043 180
- US-A1- 2008 027 194
- US-A1- 2009 069 516

## Description

### FIELD OF THE INVENTION

This invention relates to metathesis catalyst compounds and processes for the use thereof.

### BACKGROUND OF THE INVENTION

The cross-metathesis of ethylene and internal olefin to produce alpha-olefins is generally referred to as ethenolysis. Olefins Conversion Technology™, based upon the Phillips Triolefin Process, is an example of an ethenolysis reaction converting ethylene and 2-butene into propylene. These processes uses heterogeneous catalysts, such as tungsten and rhenium oxides, which have not proven effective for internal olefins containing functional groups such as cis-methyl oleate, a fatty acid methyl ester.

Methods for the production of polyalpha-olefins are typically multi-step processes that often create unwanted by-products and waste of reactants and energy. Full range linear alpha-olefins plants are petroleum-based, are inefficient and result in mixtures of oligomerization products that typically yield Schulz-Flory distributions producing large quantities of undesirable materials. In recent years there have been new technologies implemented to produce "on purpose" linear alpha-olefins such 1-hexene and 1-octene through chromium-based selective ethylene trimerization or tetramerization catalysts. Alternatively, 1-octene has been produced via the telomerization of butadiene and methanol. Similar strategies are not currently available for the production of 1-decene.

1-Decene is a co-product in the cross-metathesis of ethylene and methyl oleate. Alkyl oleates are fatty acid esters that can be major components in biodiesel produced by the transesterification of alcohol and vegetable oils. Vegetable oils containing at least one site of unsaturation include canola, soybean, palm, peanut, mustard, sunflower, tung, tall, perilla, grapeseed, rapeseed, linseed, safflower, pumpkin corn and many other oils extracted from plant seeds. Alkyl erucates similarly are fatty acid esters that can be major components in biodiesel. Useful biodiesel compositions are those which typically have high concentrations of oleate and erucate esters. These fatty acid esters preferably have one site of unsaturation such that cross-metathesis with ethylene yields 1-decene as a co-product.

Biodiesel is a fuel prepared from renewable sources, such as plant oils or animal fats. To produce biodiesel, triacylglycerides ("TAG"), the major compound in plant oils and animal fats, are converted to fatty acid alkyl esters ("FAAE," i.e., biodiesel) and glycerol via reaction with an alcohol in the presence of a base, acid, or enzyme catalyst. Biodiesel fuel can be used in diesel engines, either alone or in a blend with petroleum-based diesel, or can be further modified to produce other chemical products.

Cross-metathesis catalysts reported thus far for the ethenolysis of methyl oleate are typically ruthenium-based catalysts bearing phosphine or carbene ligands. Dow researchers in 2004 achieved catalysts turnovers of approximately 15,000 using the 1^{st} generation Grubb's catalyst, bis(tricyclohexylphosphine)benzylidene ruthenium(IV) dichloride, (Organometallics 2004, 23, 2027). WO 2008/046106 discloses ruthenium-based catalysts for olefin metathesis of fatty acid methyl esters. Researchers at Materia, Inc. have reported turnover numbers up to 35,000 using a ruthenium catalyst containing a cyclic alkyl amino carbene ligand, (WO 2008/010961). These turnovers were obtained with a catalyst reportedly too expensive for industrial consideration due to high costs associated with the catalysts being derived from a low yielding synthesis (See Final Technical Report entitled "Platform Chemicals from an Oilseed Biorefinery" grant number DE-FG36-04G014016 awarded by the Department of Energy). In order to obtain an economically viable process for 1-decene production via the cross-metathesis of ethylene and biodiesel or vegetable oils, higher activity catalysts must be discovered. Thus there is a need for higher activity processes that produce desired products and co-products in commercially desirable ratios.

Likewise, there is a need to develop a means to provide linear alpha-olefins (particularly high yields of linear alpha-olefins) by metathesis reactions, particularly reactions with good conversion, preferably under mild reaction conditions is a minimal number of steps. The instant invention's metathesis catalyst provides both a commercially economical and an "atom-economical" route to linear alpha-olefins.

Specifically, the instant inventors have found that the combination of a cyclic alkyl amino carbene ligand attached to ruthenium with a chelating benzylidene ligand containing an electron withdrawing group yielded a catalyst that is both highly active and very selective towards the ethenolysis of methyl oleate yielding 1-decene and methyl-9-decenoate.

### SUMMARY OF THE INVENTION.

This invention relates to a process comprising contacting a seed oil or derivative thereof (and optional alkene) with an olefin metathesis catalyst under conditions which yield an alpha-olefin.

This invention relates to a process comprising contacting a triacylglyceride or derivative thereof (and optional alkene) with an olefin metathesis catalyst under conditions which yield an alpha-olefin.

The novel process of this invention employs a novel catalyst comprising the combination of a cyclic alkyl amino carbene ligand and a benzylidene ligand containing an electron withdrawing substituent on the aromatic ring of the benzylidene both attached to a Group 8 metal, such as a ruthenium atom.

This invention further relates to a metathesis catalyst is represented by the formula: 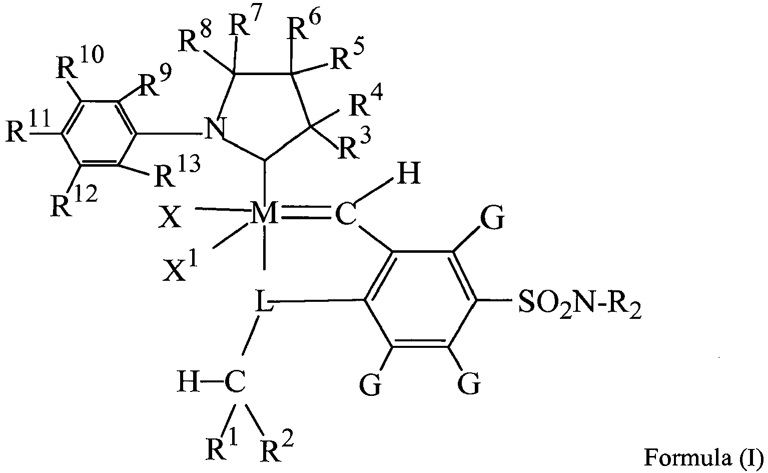 where:
M is a Group 8 metal;
X and X¹ are, independently, any anionic ligand, or X and X¹ may be joined to form a dianionic group and may form single ring of up to 30 non-hydrogen atoms or a multinuclear ring system of up to 30 non-hydrogen atoms;
Lis O or S;
R is hydrogen or a C₁ to C₃₀ hydrocarbyl or substituted hydrocarbyl;
R¹, R², R³, R⁴, R⁵, R⁶, R⁷, and R⁸ are, independently, hydrogen or a C₁ to C₃₀ hydrocarbyl or substituted hydrocarbyl;
each R⁹ and R¹³ are, independently, hydrogen or a C₁ to C₃₀ hydrocarbyl or substituted hydrocarbyl;
R¹⁰, R¹¹, R¹² are, independently, hydrogen or a C₁ to C₃₀ hydrocarbyl or substituted hydrocarbyl;
each G, is, independently, hydrogen, halogen or C₁ to C₃₀ substituted or unsubstituted hydrocarbyl,
where any two adjacent R groups may form a single ring of up to 8 non-hydrogen atoms or a multinuclear ring system of up to 30 non-hydrogen atoms.

### DETAILED DESCRIPTION

This invention relates to a process comprising contacting a seed oil or derivative thereof (and optional alkene) with an olefin metathesis catalyst under conditions which yield an alpha-olefin. Typically the seed oil is esterified or transesterified with an alcohol prior to contacting with the olefin metathesis catalyst.

This invention relates to a process comprising contacting a triacylglyceride or a derivative thereof with an optional alkene (such as ethylene) and an olefin metathesis catalyst under conditions which yield an alpha-olefin, typically yielding a linear alpha-olefin (such as1-decene, 1-heptene, and/or 1-butene) and an ester or acid functionalized olefin.

This invention further relates to a process for producing alpha-olefins (preferably linear alpha-olefins) comprising contacting a triacylglyceride with an alcohol (such as methanol) to produce a fatty acid alkyl ester and thereafter contacting the fatty acid alkyl ester with an olefin metathesis catalyst (and optional alkene, such as ethylene) under conditions which yield an alpha-olefin (preferably a linear alpha-olefin, preferably 1-decene, 1-heptene, and/or 1-butene) and an ester or acid functionalized olefin.

This invention further relates to a process for producing alpha-olefins (preferably linear alpha-olefins) comprising contacting a triacylglyceride with water and or an alkaline reactant (such as sodium hydroxide) to produce a fatty acid and thereafter contacting the fatty acid with an olefin metathesis catalyst (and optional alkene, such as ethylene) under conditions which yield an alpha-olefin (preferably a linear alpha-olefin, preferably 1-decene, 1-heptene, and/or 1-butene) and an ester or acid functionalized olefin.

This invention further relates to contacting unsaturated fatty acid with an alkene (such as ethylene) in the presence of an olefin metathesis catalyst under conditions which yield an alpha-olefin (preferably a linear alpha-olefin, preferably 1-decene, 1-heptene, and/or 1-butene) and an ester or acid functionalized olefin.

This invention further relates to contacting an unsaturated fatty acid ester with a alkene (such as ethylene) in the presence of an olefin metathesis catalyst under conditions which yield an alpha-olefin (preferably a linear alpha-olefin, preferably 1-decene, 1-heptene, and/or 1-butene) and an ester or acid functionalized olefin.

This invention further relates to contacting an unsaturated fatty acid alkyl ester with a alkene (such as ethylene) in the presence of an olefin metathesis catalyst under conditions which yield an alpha-olefin (preferably a linear alpha-olefin, preferably 1-decene, 1-heptene, and/or 1-butene) and an ester or acid functionalized olefin.

This invention also relates to a process to produce alpha olefin (preferably linear alpha olefin, preferably 1-decene, 1-heptene and or 1-butene) comprising contacting a metathesis catalyst with an alkene (preferably ethylene), and one or more fatty acid esters (preferably fatty acid methyl esters, preferably methyl oleate).

In a preferred embodiment, this relates to a process to produce alpha olefin (preferably linear alpha olefin, preferably 1-decene, 1-heptene and or 1-butene) comprising contacting a metathesis catalyst with an alkene (preferably ethylene), and one or more fatty acid esters (preferably fatty acid methyl esters, preferably methyl oleate) derived from biodiesel.

In a preferred embodiment, the olefin metathesis catalysts described herein may be combined directly with triacylglycerides, biodiesel, fatty acids, fatty acid esters and/or fatty acid alkyl esters to produce alpha-olefins, preferably linear alpha olefins, preferably C₄ to C₂₄ alpha-olefins, preferably linear alpha-olefins, such as 1-decene, 1-heptene and or 1-butene.

In a preferred embodiment, a mixture of one or more triacylglyceride, biodiesel, fatty acids, fatty acid esters and/or fatty acid alkyl esters are used to produce alpha-olefins, preferably linear alpha olefins, preferably C₄ to C₂₄ alpha-olefins, preferably C₄ to C₂₄ linear alpha-olefins. In a preferred embodiment a mixture of alpha olefins, preferably linear alpha olefins, preferably 1-decene, 1-heptene and or 1-butene are produced.

### Process

In a preferred embodiment, the metathesis catalysts described herein may be combined directly with seed oils, triacylglyceride, biodiesel, fatty acids, fatty acid esters and/or fatty acid alkyl esters ("feed materials") to produce alpha-olefins, preferably linear alpha olefins, preferably C₄ to C₂₄ alpha-olefins, preferably C₄ to C₂₄ linear alpha-olefins, such as preferably 1-decene, 1-heptene and or 1-butene.

Typically, the molar ratio of alkene to unsaturated feed material (such as unsaturated fatty acid or fatty acid ester) is greater than about 0.8/1.0, preferably, greater than about 0.9/1.0. Typically, the molar ratio of alkene to feed material (such as unsaturated fatty acid or fatty acid ester) is less than about 3.0/1.0, preferably, less than about 2.0/1.0. Depending upon the specific reagents, other molar ratios may also be suitable. With ethylene, for example, a significantly higher molar ratio can be used, because the self-metathesis of ethylene produces only ethylene again; no undesirable co-product olefins are formed. Accordingly, the molar ratio of ethylene to feed material (such as unsaturated fatty acid or fatty acid ester) may range from greater than about 0.8/1 to typically less than about 20/1.

The quantity of metathesis catalyst that is employed in the process of this invention is any quantity that provides for an operable metathesis reaction. Preferably, the ratio of moles of feed material (preferably fatty acid ester and or fatty acid alkyl ester) to moles of metathesis catalyst is typically greater than about 10:1, preferably, greater than about 100:1, preferably greater than about 1000:1, preferably greater than about 10,000:1, preferably greater than about 25,000:1, preferably greater than about 50,000:1, preferably greater than about 100,000:1. Alternately, the molar ratio of feed material (preferably fatty acid ester and or fatty acid alkyl ester) to metathesis catalyst is typically less than about 10,000,000:1, preferably, less than about 1,000,000:1, and more preferably, less than about 500,000:1.

The contacting time of the reagents and catalyst in a batch reactor can be any duration, provided that the desired olefin metathesis products are obtained. Generally, the contacting time in a reactor is greater than about 5 minutes, and preferably, greater than about 10 minutes. Generally, the contacting time in a reactor is less than about 25 hours, preferably, less than about 15 hours, and more preferably, less than about 10 hours.

In a preferred embodiment, the reactants (for example, metathesis catalyst; feed materials; optional alkene, optional alcohol, optional water, etc) are combined in a reaction vessel at a temperature of 20 to 200°C (preferably 30 to 100°C, preferably 40 to 60°C) and an alkene (such as ethylene) pressure of 0.1 to 1000 psi (690 Pa to 690,000 Pa) (preferably 20 to 400 psi (138,000 to 2,758,000 Pa), preferably 50 to 250 psi (345,000 to 1,724,000 Pa)), if the alkene is present, for a residence time of 0.5 seconds to 48 hours (preferably 0.25 to 5 hours, preferably 30 minutes to 2 hours).

In a preferred embodiment, from about 0.005 nmoles to about 500 nmoles, preferably from about 0.1 to about 250 nmoles, and most preferably from about 1 to about 50 nmoles of the metathesis catalyst are charged to the reactor per 3 mmoles of feed material (such as TAGs, biodiesel, fatty acids, fatty acid esters and/or fatty acid alkyl esters or mixtures thereof, preferably fatty acid esters) charged.

In a preferred embodiment, the alkane and an unsaturated fatty acid ester or unsaturated fatty acid are co-metathesized to form first and second product olefins, preferably, a reduced chain first product alpha-olefin and a second product reduced chain terminal ester or acid-functionalized alpha-olefin. As a preferred example, the metathesis of methyloleate with ethylene will yield co-metathesis products of 1-decene and methyl-9-decenoate. Both products are alpha-olefins; the decenoate also terminates in an ester moiety at the opposite end of the chain from the carbon-carbon double bond. In addition to the desired products, the methyloleate may self-metathesize to produce small amounts of 9-octadecene, a less desirable product, and dimethyloctadec-9-enoate, CH₃O(O)C(CH₂)₇CH=CH(CH₂)₇C(O)OCH₃, a second less desirable product.

In the process of this invention, the conversion of feed material (preferably fatty acid ester and or fatty acid alkyl ester) can vary widely depending upon the specific reagent olefins, the specific catalyst, and specific process conditions employed. For the purpose of this invention, "conversion" is defined as the mole percentage of feed material that is converted or reacted to products. Typically, the conversion of feed material (preferably fatty acid ester and or fatty acid alkyl ester) is greater than about 50 mole percent, preferably, greater than about 60 mole percent, and more preferably, greater than about 70 mole percent.

In the process of this invention, the yields of first product olefin and ester or acid-functionalized second product olefin can also vary depending upon the specific reagent olefins, catalyst, and process conditions employed. For the purposes of this invention "yield" will be defined as the mole percentage of product olefin formed relative to the initial moles of feed material (such as fatty acid ester and or fatty acid alkyl ester) in the feed. Typically, the yield of alpha-olefin will be greater than about 35 mole percent, preferably, greater than about 50 mole percent. Typically, the yield of ester or acid-functionalized alpha-olefin will be greater than about 35 mole percent, preferably, greater than about 50 mole percent.

In a preferred embodiment, the process is typically a solution process, although it may be a bulk or high pressure process. Homogeneous processes are preferred. (A homogeneous process is defined to be a process where at least 90 wt% of the product is soluble in the reaction media.) A bulk homogeneous process is particularly preferred. (A bulk process is defined to be a process where reactant concentration in all feeds to the reactor is 70 volume % or more.) Alternately no solvent or diluent is present or added in the reaction medium, (except for the small amounts used as the carrier for the catalyst or other additives, or amounts typically found with the reactants; e.g., propane in propylene).

Suitable diluents/solvents for the process include non-coordinating, inert liquids. Examples include straight and branched-chain hydrocarbons such as isobutane, butane, pentane, isopentane, hexanes, isohexane, heptane, octane, dodecane, and mixtures thereof; cyclic and alicyclic hydrocarbons such as cyclohexane, cycloheptane, methylcyclohexane, methylcycloheptane, and mixtures thereof such as can be found commercially (Isopar™); perhalogenated hydrocarbons such as perfluorinated C₄₋₁₀ alkanes, chlorobenzene, and aromatic and alkylsubstituted aromatic compounds such as benzene, toluene, mesitylene, and xylene. Suitable diluents/solvents also include aromatic hydrocarbons, such as toluene or xylenes, and chlorinated solvents, such as dichloromethane. In a preferred embodiment, the feed concentration for the process is 60 volume % solvent or less, preferably 40 volume % or less, preferably 20 volume % or less.

The process may be batch, semi-batch or continuous. As used herein, the term continuous means a system that operates without interruption or cessation. For example, a continuous process to produce a polymer would be one where the reactants are continually introduced into one or more reactors and polymer product is continually withdrawn.

Useful reaction vessels include reactors (including continuous stirred tank reactors, batch reactors, reactive extruder, pipe or pump.

If the process is conducted in a continuous flow reactor, then the weight hourly space velocity, given in units of grams feed material (preferably fatty acid ester and or fatty acid alkyl ester) per gram catalyst per hour (h⁻¹), will determine the relative quantities of feed material to catalyst employed, as well as the residence time in the reactor of the unsaturated starting compound. In a flow reactor, the weight hourly space velocity of the unsaturated feed material (preferably fatty acid ester and or fatty acid alkyl ester) is typically greater than about 0.04 g feed material (preferably fatty acid ester and or fatty acid alkyl ester) per g catalyst per hour (h⁻¹), and preferably, greater than about 0.1 h⁻¹. In a flow reactor, the weight hourly space velocity of the feed material (preferably fatty acid ester and or fatty acid alkyl ester) is typically less than about 100 h⁻¹, and preferably, less than about 20 h⁻¹.

In a preferred embodiment, the productivity of the process is at least 200 g of linear alpha-olefin (such as decene-1) per mmol of catalyst per hour, preferably at least 5000 g/mmol/hour, preferably at least 10,000 g/mmol/hr, preferably at least 300,000 g/mmol/hr.

In a preferred embodiment, the selectivity of the process is at least 20 wt% linear alpha-olefin, based upon the weight to the material exiting the reactor, preferably at least 25%, preferably at least 30%, preferably at least 35%.

In a preferred embodiment, the turnover number (TON), defined as the moles of alpha olefin formed per mol of catalyst, of the process is at least 10,000, preferably at least 50,000, preferably at least 100,000, preferably at least 1,000,000.

In a preferred embodiment, the yield (when converting unsaturated fatty acids, unsaturated fatty acid esters, unsaturated fatty acid alkyl esters or mixtures thereof), defined as the moles of alpha olefin formed per mol of unsaturated fatty acids, unsaturated fatty acid esters, unsaturated fatty acid alkyl esters or mixtures thereof introduced into the reactor, is 30% or more, preferably 40% or more, preferably 45% or more, preferably 50% or more, preferably 55% or more, preferably 60% or more.

In a preferred embodiment, the yield for reactions (when converting TAGs as represented in the formula below), is defined as the moles of alpha olefin formed divided by (the moles of unsaturated R^{a} + moles of unsaturated R^{b} + moles of unsaturated R^{c}) introduced into the reactor is 30% or more, preferably 40% or more, preferably 45% or more, preferably 50% or more, preferably 55% or more, preferably 60% or more. 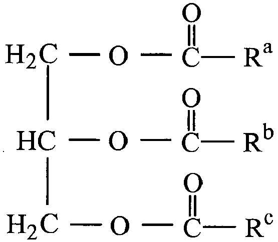 where R^{a}, R^{b} and R^{c} each, independently, represent a saturated or unsaturated hydrocarbon chain (preferably R^{a}, R^{b} and R^{c} each, independently, are a C₁₂ to C₂₈ alkyl or alkene, preferably C₁₆ to C₂₂ alkyl or alkene).

### Alkenes

Besides the feed materials, the metathesis process of this invention may require a an alkene as a reactant. The term "alkene" shall imply an organic compound containing at least one carbon-carbon double bond and typically having less than about 10 carbon atoms. The alkene may have one carbon-carbon unsaturated bond, or alternatively, two or more carbon-carbon unsaturated bonds. Since the metathesis reaction can occur at any double bond, alkenes having more than one double bond will produce more metathesis products. Accordingly, in some embodiments, it is preferred to employ an alkene having only one carbon-carbon double bond. The double bond may be, without limitation, a terminal double bond or an internal double bond. The alkene may also be substituted at any position along the carbon chain with one or more substituents, provided that the one or more substituents are essentially inert with respect to the metathesis process. Suitable substituents include, without limitation, alkyl, preferably, C₁₋₆ alkyl; cycloalkyl, preferably, C₃₋₆ cycloalkyl; as well as hydroxy, ether, keto, aldehyde, and halogen functionalities. Non-limiting examples of suitable alkenes include ethylene, propylene, butene, butadiene, pentene, hexene, the various isomers thereof, as well as higher homologues thereof. Preferably, the alkene is a C₂₋₈ alkene. More preferably, the alkene is a C₂₋₆ alkene, even more preferably, a C₂₋₄ alkene, and most preferably, ethylene.

Useful alkenes include those represented by the formula: R*-HC=CH-R*, wherein each R* is independently, hydrogen or a C₁ to C₂₀ hydrocarbyl, preferably hydrogen or a C₁ to C₆ hydrocarbyl, preferably hydrogen, methyl, ethyl, propyl or butyl, more preferably R* is hydrogen. In a preferred embodiment, both R* are the same, preferably both R*are hydrogen. Ethylene, propylene, butene, pentene, hexene, octene and nonene (preferably ethylene) are alkenes useful herein.

For purposes of this invention and the claims thereto, the term lower olefin means an alkene represented by the formula: R*-HC=CH-R*, wherein each R* is independently, hydrogen or a C₁ to C₆ hydrocarbyl, preferably hydrogen or a C₁ to C₃ hydrocarbyl, preferably hydrogen, methyl, ethyl, propyl or butyl, more preferably R* is hydrogen. In a preferred embodiment, both R* are the same, preferably both R*are hydrogen. Ethylene, propylene, butene, pentene, hexene, and octene (preferably ethylene) are lower olefins useful herein.

### Triacylglycerides

Triacylglycerides (TAGs), also called triglycerides, are a naturally occurring ester of three fatty acids and glycerol that is the chief constituent of natural fats and oils. The three fatty acids can be all different, all the same, or only two the same, they can be saturated or unsaturated fatty acids, and the saturated fatty acids may have one or multiple unsaturations. Chain lengths of the fatty acids in naturally occurring triacylglycerides can be of varying lengths but 16, 18 and 20 carbons are the most common. Natural fatty acids found in plants and animals are typically composed only of even numbers of carbon atoms due to the way they are bio-synthesized. Most natural fats contain a complex mixture of individual triglycerides and because of this, they melt over a broad range of temperatures.

Biodiesel is a mono-alkyl ester derived from the processing of vegetable oils and alcohols. The processing is typically carried out by an esterification reaction mechanism, and typically is performed in an excess of alcohol to maximize conversion. Esterification can refer to direct esterification, such as between a free fatty acid and an alcohol, as well as transesterification, such as between an ester and an alcohol. While vegetable oil and alcohols are commonly employed as reactants in esterification reactions, a fatty acid source such as free fatty acids, soaps, esters, glycerides (mono-, di- tri-), phospholipids, lysophospholipids, or amides and a monohydric alcohol source, such as an alcohol or an ester, can be esterified. In addition, various combinations of these reagents can be employed in an esterification reaction.

Vegetable oils include triglycerides and neutral fats, such as triacylglyderides, the main energy storage form of fat in animals and plants. These typically have the chemical structure: 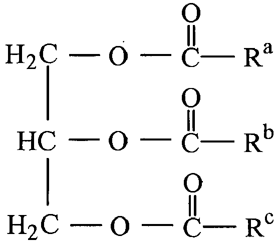 where R^{a}, R^{b} and R^{c} each, independently, represent a saturated or non-saturated hydrocarbon chain (preferably R^{a}, R^{b} and R^{c} each, independently, are a C₁₂ to C₂₈ alkyl or alkene, preferably C₁₆ to C₂₂ alkyl or alkene). Different vegetable oils have different fatty acid profiles, with the same or different fatty acids occurring on a single glycerol. For example, an oil can have linoleic, oleic, and stearic acids attached to the same glycerol, with each of R^{a}, R^{b} and R^{c} representing one of these three fatty acids. In another example, there can be two oleic acids and one stearic acid attached to the same glycerol, each of R^{a}, R^{b} and R^{c} representing one of these fatty acids. A particularly useful triglyceride consists of three fatty acids (e.g., saturated fatty acids of general structure of CH₃(CH₂)ₙCOOH, wherein n is typically an integer of from 4 to 28 or higher) attached to a glycerol (C₃H₅(OH)₃) backbone by ester linkages. In the esterification process, vegetable oils and short chain alcohols are reacted to form mono-alkyl esters of the fatty acid and glycerol (also referred to as glycerin). When the alcohol used is methanol (CH₃OH), a methyl ester is created with the general form CH₃(CH₂)ₙCOOCH₃ for saturated fatty acids. Typically, but not always, the length of the carbon backbone chain is from 12 to 24 carbon atoms.

The esterification process can be catalyzed or non-catalyzed. Catalyzed processes are categorized into chemical and enzyme based processes. Chemical catalytic methods can employ acid and/or base catalyst mechanisms. The catalysts can be homogeneous and/or heterogeneous catalysts. Homogeneous catalysts are typically liquid phase mixtures, whereas heterogeneous catalysts are solid phase catalysts mixed with the liquid phase reactants, oils and alcohols.

The fatty acid rich material useful in the processes described herein can be derived from plant, animal, microbial, or other sources (feed oil). Preferred feed oils include vegetable oils such as corn, soy, rapeseed, canola, sunflower, palm and other oils that are readily available; however, any vegetable oil or animal fat can be employed. Raw or unrefined oil can be used in certain embodiments; however, filtered and refined oils are typically preferred. Use of degummed and filtered feedstock minimizes the potential for emulsification and blockage in the reactors. Feedstock with high water content can be dried before basic catalyst processing. Feedstock with high free fatty acid content can be passed through an esterification process to reduce the free fatty acid content before the process of esterification to convert fatty acid glycerides to monoalkyl esters. The reduction of free fatty acids and the conversion of fatty acid glycerides can also in the same processing step. Feedstock containing other organic compounds (such as hexane, heptane, isohexane, etc.) can typically be processed without significant modifications to the reactor. Other materials containing fatty acid glycerides or other fatty acid esters can also be employed, including phospholipids, lysophospholipids, and fatty acid wax esters. The fatty acid rich material useful in the processes described herein typically includes a mixture of fatty acids. For example, the fatty acid profiles of several potential feedstocks are shown in Table 1. The feed oil can also include a mixture of fatty acid glycerides from different sources. The free fatty acid content of useful vegetable oils is preferably about 0.1 wt% or less when employed in a basic homogeneous catalyst esterification reaction. Higher levels can be utilized as well, and levels up to about 3 wt%, or even as high as 15 wt% or more can typically be tolerated.

**TABLE 1**

| Fatty Acid Profile of Several Typical Feed Oils | | | | |
|---|---|---|---|---|
| Fatty Acid | Palm Oil | Soy Oil | Hi Oleic Rapeseed | Yellow Grease |
| | 0 wt% | 0 wt% | 0 wt% | 0 wt% |
| C6:0 | 0 wt% | 0 wt% | 0 wt% | 0 wt% |
| C8:0 | 0 wt% | 0 wt% | 0 wt% | 0 wt% |
| C10:0 | 0 wt% | 0 wt% | 0 wt% | 0 wt% |
| C12:0 | 0 wt% | 0 wt% | 0 wt% | 0 wt% |
| C14:0 | 1 wt% | 0 wt% | 0 wt% | 2 wt% |
| C16:0 | 44 wt% | 7 wt% | 4 wt% | 23 wt% |
| C18:0 | 5 wt% | 5 wt% | 1 wt% | 13 wt% |
| C18:1 | 39 wt% | 28wt% | 60 wt% | 44 wt% |
| C18:2 | 10 wt% | 53 wt% | 21 wt% | 7 wt% |
| C18:3 | 0 wt% | 0 wt% | 13 wt% | 1 wt% |
| C20:0 | 0 wt% | 0 wt% | 0 wt% | 0 wt% |
| C22:1 | 0 wt% | 0 wt% | 0 wt% | 0 wt% |
| Misc. | 1 wt% | 8 wt% | 0 wt% | 9 wt% |
| Total | 100 wt% | 100 wt% | 100 wt% | 100 wt% |

### Alcohol (also referred to as Alkanols)

The alcohol used herein can be any monohydric, dihydric, or polyhydric alcohol that is capable of condensing with the feed material (such as the unsaturated fatty acid) to form the corresponding unsaturated ester ( such as the fatty acid ester). Typically, the alcohol contains at least one carbon atom. Typically, the alcohol contains less than about 20 carbon atoms, preferably, less than about 12 carbon atoms, and more preferably, less than about 8 carbon atoms. The carbon atoms may be arranged in a straight-chain or branched structure, and may be substituted with a variety of substituents, such as those previously disclosed hereinabove in connection with the fatty acid, including the aforementioned alkyl, cycloalkyl, monocyclic aromatic, arylalkyl, alkylaryl, hydroxyl, halogen, ether, ester, aldehyde and keto substituents. Preferably, the alcohol is a straight-chain or branched C₁₋₁₂ alkanol. A preferred alcohol is the trihydric alcohol glycerol, the fatty acid esters of which are known as "glycerides." Other preferred alcohols include methanol and ethanol.

Preferably, the alcohol employed in the esterification and/or transesterification reactions is preferably a low molecular weight monohydric alcohol such as methanol, ethanol, 1-propanol, 2-propanol, 1-butanol, 2-butanol, or t-butanol. The alcohol is preferably anhydrous; however, a small amount of water in the alcohol may be present (e.g., less than about 2 wt%, preferably less than about 1 wt%, and most preferably less than about 0.5 wt%; however in certain embodiments higher amounts can be tolerated). Acid esterification reactions are more tolerant of the presence of small amounts of water in the alcohol than are basic transesterification reactions. While specific monohydric alcohols are discussed herein with reference to certain embodiments and examples, the preferred embodiments are not limited to such specific monohydric alcohols. Other suitable monohydric alcohols can also be employed in the preferred embodiments.

### Transesterification /Esterification Reactions

The conversion of TAGs to fatty acid alkyl esters ("FAAE") through transesterification of the TAG typically involves forming a reactant stream, which includes TAG (e.g., at least about 75 wt%), alkanol (e.g., about 5 to 20 wt%), a transesterification catalyst (e.g., about 0.05 to 1 wt%), and optionally, glycerol (typically up to about 10 wt%). Suitable alkanols may include C1-C6 alkanols and commonly may include methanol, ethanol, or mixtures thereof. Suitable transesterification catalysts may include alkali metal alkoxides having from 1 to 6 carbon atoms and commonly may include alkali metal methoxide, such as sodium methoxide and/or potassium methoxide. The basic catalyst is desirably selected such that the alkali metal alkoxide may suitably contain an alkoxide group which is the counterpart of the alkanol employed in the reaction stream (e.g., a combination of methanol and an alkali metal methoxide such as sodium methoxide and/or potassium methoxide). The reactant stream may suitably include about 0.05 to 0.3 wt% sodium methoxide, at least about 75 wt% triacylglyceride, about 1 to 7 wt% glycerol, and at least about 10 wt% methanol. In some embodiments, the reactant stream may desirably include about 0.05 to 0.25 wt% sodium methoxide, at least about 75 wt% triacylglyceride, about 2 to 5 wt% glycerol, and about 10 to 15 wt% methanol.

The rate and extent of reaction for esterification of the fatty acid glycerides or other fatty acid derivates with monohydric alcohol in the presence of a catalyst depends upon factors including but not limited to the concentration of the reagents, the concentration and type of catalyst, and the temperature and pressure conditions, and time of reaction. The reaction generally proceeds at temperatures above about 50°C, preferably at temperatures above 65°C; however, the catalyst selected or the amount of catalyst employed can affect this temperature to some extent. Higher temperatures generally result in faster reaction rates. However, the use of very high temperatures, such as those in excess of about 300°C, or even those in excess of 250°C, can lead to increased generation of side products, which can be undesirable as their presence can increase downstream purification costs. Higher temperatures can be advantageously employed, however, e.g., in situations where the side products do not present an issue.

The reaction temperature can be achieved by preheating one or more of the feed materials or by heating a mixture of the feed materials. Heating can be achieved using apparatus known in the art e.g., heat exchangers, jacketed vessels, submerged coils, and the like. While specific temperatures and methods of obtaining the specific temperatures are discussed herein with reference to certain embodiments and examples, the preferred embodiments are not limited to such specific temperatures and methods of obtaining the specific temperatures. Other temperatures and methods of obtaining temperatures can also be employed in the preferred embodiments.

The amount of alcohol employed in the reaction is preferably in excess of the amount of fatty acid present on a molar basis. The fatty acid can be free or combined, such as to alcohol, glycol or glycerol, with up to three fatty acid moieties being attached to a glycerol. Additional amounts of alcohol above stoichiometric provide the advantage of assisting in driving the equilibrium of the reaction to produce more of the fatty acid ester product. However, greater excesses of alcohol can result in greater processing costs and larger capital investment for the larger volumes of reagents employed in the process, as well as greater energy costs associated with recovering, purifying, and recycling this excess alcohol. Accordingly, it is generally preferred to employ an amount of alcohol yielding a molar ratio of alcohol to fatty acid of from about 15:1 to about 1:1 (stoichiometric), and more preferably from about 4:1 to about 2:1; however, the process can operate over a much wider range of alcohol to fatty acid ratios, with nonreacted materials subjected to recycling or other processing steps. Generally, lower relative levels of alcohol to fatty acid result in decreased yield and higher relative levels of alcohol levels to fatty acid result in increased capital and operating expense. Some instances of operation at ratios of alcohol to fatty acid over a wider range include when first starting up the process or shutting down the process, when balancing the throughput of the reactor to other processing steps or other processing facilities, such as one that produces alcohol or utilizes a side stream, or when process upsets occur. When a molar ratio of 2:1 methanol to fatty acid is employed and a sodium hydroxide concentration of about 0.5 wt% of the total reaction mixture is employed, the ratio of sodium hydroxide to methanol is about 2 wt% entering the reactor and about 4 wt% at the exit because about half of the alcohol is consumed in the esterification reaction.

Similarly, higher amounts of catalyst generally result in faster reactions. However, higher amounts of catalyst can lead to higher downstream separation costs and a different profile of side reaction products. The amount of homogeneous catalyst is preferably from about 0.2 wt% to about 1.0 wt% of the reaction mixture when the catalyst is sodium hydroxide; at typical concentration of 0.5 wt% when a 2:1 molar ratio of methanol to fatty acid is used; however, in certain embodiments higher or lower amounts can be employed. The amount of catalyst employed can also vary depending upon the nature of the catalyst, feed materials, operating conditions, and other factors. Specifically, the temperature, pressure, free fatty acid content of the feed, and degree of mixing can change the amount of catalyst preferably employed. While specific catalyst amounts are discussed herein with reference to certain embodiments and examples, the preferred embodiments are not limited to such specific catalyst amounts. Other suitable catalyst amounts can also be employed in the preferred embodiments.

The esterification reaction can be performed batchwise, such as in a stirred tank, or it can be performed continuously, such as in a continuous stirred tank reactor (CSTR) or a plug flow reactor (PFR). When operated in continuous mode, a series of continuous reactors (including CSTRs, PFRs, or combinations thereof) can advantageously operate in series. Alternatively, batch reactors can be arranged in parallel and/or series.

When the reactor is operated in a continuous fashion, one or more of the feed materials is preferably metered into the process. Various techniques for metering can be employed (e.g., metering pumps, positive displacement pumps, control valves, flow meters, and the like). While specific types of reactors are discussed herein with reference to certain embodiments and examples, the preferred embodiments are not limited to such specific reactors. Other suitable types of reactors can also be employed in the preferred embodiments.

### Fatty Acids and Fatty Acid Esters

Fatty acids are carboxylic acids with a saturated or unsaturated aliphatic tails that are found naturally in many different fats and oils. Any unsaturated fatty acid can be suitably employed in the process of this invention, provided that the unsaturated fatty acid can be metathesized in the manner disclosed herein. An unsaturated fatty acid comprises a long carbon chain containing at least one carbon-carbon double bond and terminating in a carboxylic acid group. Typically, the unsaturated fatty acid will contain greater than about 8 carbon atoms, preferably, greater than about 10 carbon atoms, and more preferably, greater than about 12 carbon atoms. Typically, the unsaturated fatty acid will contain less than about 50 carbon atoms, preferably, less than about 35 carbon atoms, and more preferably, less than about 25 carbon atoms. At least one carbon-carbon double bond is present along the carbon chain, this double bond usually occurring about the middle of the chain, but not necessarily. The carbon-carbon double bond may also occur at any other internal location along the chain. A terminal carbon-carbon double bond, at the opposite end of the carbon chain relative to the terminal carboxylic acid group, is also suitably employed, although terminal carbon-carbon double bonds occur less commonly in fatty acids. Unsaturated fatty acids containing the terminal carboxylic acid functionality and two or more carbon-carbon double bonds may also be suitably employed in the process of this invention. Since metathesis can occur at any of the carbon-carbon double bonds, a fatty acid having more than one double bond may produce a variety of metathesis products. The unsaturated fatty acid may be straight or branched and substituted along the fatty acid chain with one or more substituents, provided that the one or more substituents are substantially inert with respect to the metathesis process. Non-limiting examples of suitable substituents include alkyl moieties, preferably C₁₋₁₀ alkyl moieties, including, for example, methyl, ethyl, propyl, butyl, and the like; cycloalkyl moieties, preferably, C₄₋₈ cycloalkyl moieties, including for example, cyclopentyl and cyclohexyl; monocyclic aromatic moieties, preferably, C₆ aromatic moieties, that is, phenyl; arylalkyl moieties, preferably, C₇₋₁₆ arylalkyl moieties, including, for example, benzyl; and alkylaryl moieties, preferably, C₇₋₁₆ alkylaryl moieties, including, for example, tolyl, ethylphenyl, xylyl, and the like; as well as hydroxyl, ether, keto, aldehyde, and halide, preferably, chloro and bromo, functionalities.

Non-limiting examples of suitable unsaturated fatty acids include 3-hexenoic (hydrosorbic), trans-2-heptenoic, 2-octenoic, 2-nonenoic, cis-and trans-4-decenoic, 9-decenoic (caproleic), 10-undecenoic (undecylenic), trans-3-dodecenoic (linderic), tridecenoic, cis-9-tetradeceonic (myristoleic), pentadecenoic, cis-9-hexadecenoic (cis-9-palmitoelic), trans-9-hexadecenoic (trans-9-palmitoleic), 9-heptadecenoic, cis-6-octadecenoic (petroselinic), trans-6-octadecenoic (petroselaidic), cis-9-octadecenoic (oleic), trans-9-octadecenoic (elaidic), cis-11-octadecenoic, trans-11-octadecenoic (vaccenic), cis-5-eicosenoic, cis-9-eicosenoic (godoleic), cis-11-docosenoic (cetoleic), cis-13-docosenoic (erucic), trans-13-docosenoic (brassidic), cis-15-tetracosenoic (selacholeic), cis-17-hexacosenoic (ximenic), and cis-21-triacontenoic (lumequeic) acids, as well as 2,4-hexadienoic (sorbic), cis-9-cis-12-octadecadienoic (linoleic), cis-9-cis-12-cis-15-octadecatrienoic (linolenic), eleostearic, 12-hydroxy-cis-9-octadecenoic (ricinoleic), and like acids. Oleic acid is most preferred. Unsaturated fatty acids can be obtained commercially or synthesized by saponifying fatty acid esters, this method being known to those skilled in the art.

Fatty acid esters are formed by condensation of a fatty acid and an alcohol. Fatty acid alkyl esters are fatty acids where the hydrogen of the -OH of the acid group is replaced by a hydrocarbyl group, typically a C₁ to C₃₀ alkyl group, preferably a C₁ to C₂₀ alkyl.

Fatty acid alkyl esters are fatty acids where the hydrogen of the -OH of the acid group is replaced by an alkyl group. Fatty acid alkyl esters useful herein are typically represented by the formula: R^-C(O)-O-R*, where R^ is a C₁ to C₁₀₀ hydrocarbyl group, preferably a C₆ to C₂₂ group, preferably a C₆ to C₁₄ 1-alkene group, and R* is an alkyl group, preferably a C₁ to C₂₀ alkyl group, preferably methyl, ethyl, butyl, pentyl and hexyl. Preferred fatty acid alkyl esters useful herein are typically represented by the formula: R^-CH₂=CH₂-R^-C(O)-O-R*, where each R^ is, independently a C₁ to C₁₀₀ alkyl group, preferably a C₆ to C₂₀, preferably a C₈ to C₁₄ alkyl group, preferably a C₉ group and R* is an alkyl group, preferably a C₁ to C₂₀ alkyl group, preferably methyl, ethyl, butyl, pentyl and hexyl. Particularly preferred fatty acid alkyl esters useful herein are represented by the formula:

CH₃-(CH₂)ₙ-C=C-(CH₂)ₘ-C(O)-O-R*,

where and R* is an alkyl group, preferably a C1 to C20 alkyl group, preferably methyl, ethyl, butyl pentyl and hexyl, m and n are, independently 1, 2, 3, 4, 5, 6, 7, 8, 9, 10, 11, 12, 13, 14, 15, or 16, preferably 5, 7, 9, or 11, preferably 7.

Fatty acid methyl esters are fatty acids where the hydrogen of the -OH of the acid group is replaced by methyl group. Fatty acid methyl esters useful herein are typically represented by the formula: R^-C(O)-O-CH₃, where R^ is a C₁ to C₁₀₀ hydrocarbyl group, preferably a C₆ to C₂₂ group, preferably a C₆ to C₁₄ 1-alkene group. Preferred fatty acid methyl esters useful herein are typically represented by the formula: R^-CH₂=CH₂-R^-C(O)-O-CH₃, where each R^ is, independently a C₁ to C₁₀₀ alkyl group, preferably a C₆ to C₂₀, preferably a C₈ to C₁₄ alkyl group, preferably a C₉ group. Particularly preferred fatty acid methyl esters useful herein are represented by the formula: CH₃-(CH₂)n-C=C-(CH₂)m-C(O)-O-CH₃, where m and n are, independently 1, 2, 3, 4, 5, 6, 7, 8, 9, 10, 11, 12, 13, 14, 15, or 16, preferably 5, 7, 9, or 11, preferably 7.

Preferred fatty acid methyl esters include methyl palmitoleate, methyl oleate, methyl gadoleate, methyl erucate, methyl linoleate, methyl linolenate, methyl soyate, and mixtures of methyl esters derived from soybean oil, beef tallow, tall oil, animal fats, waste oils/greases, rapeseed oil, algae oil, Canola oil, palm oil, Jathropa oil, high-oleic soybean oil (e.g., 75 mole % or more, preferably 85 mole % or more, preferably 90 mole % or more), high-oleic safflower oil (e.g., 75 mole % or more, preferably 85 mole % or more, preferably 90 mole % or more), high-oleic sunflower oil (e.g., 75 mole % or more, preferably 85 mole % or more, preferably 90 mole % or more), and other plant or animal derived sources containing fatty acids.

A preferred source of fatty acid methyl esters for use herein includes TAG's and biodiesel sources. As described above, biodiesel refers to a transesterified vegetable oil or animal fat based diesel fuel containing long-chain alkyl (typically methyl, propyl, or ethyl) esters. Biodiesel is typically made by chemically reacting lipids (such as vegetable oil) with an alcohol. Biodiesel, TAG's and derivatives thereof may be used in the processes described herein. Likewise, preferred fatty acid methyl esters useful herein may be obtained by reacting canola oil, corn oil, soybean oil, beef tallow, tall oil, animal fats, waste oils/greases, rapeseed oil, algae oil, Canola oil, palm oil, Jathropa oil, high-oleic soybean oil, high-oleic safflower oil, high-oleic sunflower oil or mixtures of animal and/or vegetable fats and oils with one or more alcohols (as described above), preferably methanol.

Vegetable oils useful herein preferably contain at least one site of unsaturation and include, but are not limited to, canola, soybean, palm, peanut, mustard, sunflower, tung, tall, perilla, grapeseed, rapeseed, linseed, safflower, pumpkin corn and other oils extracted from plant seeds.

For purposes of this invention and the claims thereto the term "seed oil" refers to one or more vegetable or animal oils, such as canola oil, corn oil, soybean oil, beef tallow, tall oil, animal fats, waste oils/greases, rapeseed oil, algae oil, peanut oil, mustard oil, sunflower oil, tung oil, perilla oil, grapeseed oil, linseed oil, safflower oil, pumpkin oil, palm oil, Jathropa oil, high-oleic soybean oil, high-oleic safflower oil, high-oleic sunflower oil, mixtures of animal and/or vegetable fats and oils, castor bean oil, dehydrated castor bean oil, cucumber oil, poppyseed oil, flaxseed oil, lesquerella oil, walnut oil, cottonseed oil, meadowfoam, tuna oil, and sesame oils.

In a preferred embodiment, a combination of oils is used herein. Preferred combinations include two (three or four) or more of tall oil, palm oil, tallow, waste grease, rapeseed oil, canola oil, soy oil and algae oil. Alternate useful combinations include two (three or four) or more of soy oil, canola oil, rapeseed oil, algae oil, and tallow.

In certain embodiments processed oils, such as blown oils, are the source of fatty acids useful herein. While vegetable oils are preferred sources of fatty acids for practicing disclosed embodiments of the present process, fatty acids also are available from animal fats including, without limitation, lard and fish oils, such as sardine oil and herring oil, and the like. As noted above, in certain embodiments a desired fatty acid or fatty acid precursor is produced by a plant or animal found in nature. However, particular fatty acids or fatty acid precursors are advantageously available from genetically modified organisms, such as a genetically modified plants, particularly genetically modified algae. Such genetically modified organisms are designed to produce a desired fatty acid or fatty acid precursor biosynthetically or to produce increased amounts of such compounds.

Alkyl oleates and alkyl erucates are fatty acid esters that are often major components in biodiesel produced by the transesterification of alcohol and vegetable oils. (preferably the alkyls are a C₁ to C₃₀ alkyl group, alternately a C₁ to C₂₀ alkyl group.) Biodiesel compositions that are particularly useful in this invention are those which have high concentrations of alkyl oleate and alkyl erucate esters. These fatty acid esters preferably have one site of unsaturation such that cross-metathesis with ethylene yields 1-decene as the coproduct. Biodiesel compositions that are particularly useful are those produced from vegetable oils such as canola, rapeseed oil, palm oil, and other high oleate oil, high erucate oils. Particularly preferred vegetable oils include those having at least 50% (on a molar basis) combined oleic and erucic fatty acid chains of all fatty acid chains, preferably 60%, preferably70%, preferably 80%, preferably 90%.

In another embodiment, useful fatty acid ester containing mixtures include those having at least 50% (on a molar basis) alkyl oleate fatty acid esters, preferably 60% of alkyl oleate fatty acid esters, preferably 70% of alkyl oleate fatty acid esters, preferably 80% of alkyl oleate fatty acid esters, preferably 90% of alkyl oleate fatty acid esters.

In another embodiment, useful fatty acid ester containing mixtures include those having at least 50% (on a molar basis) alkyl erucate fatty acid esters, preferably 60% of alkyl erucate fatty acid esters, preferably 70% of alkyl erucate fatty acid esters, preferably 80% of alkyl erucate fatty acid esters, preferably 90% of alkyl erucate fatty acid esters.

In another embodiment, useful fatty acid ester containing mixtures include those having at least 50% (on a molar basis) combined oleic and erucic fatty acid esters of all fatty acid ester chains, preferably 60%, preferably 70%, preferably 80%, preferably 90%.

### Isomerization

In another embodiment, the feed material is first isomerized, then combined with a metathesis catalyst as described herein. For example, the processes disclosed herein may comprise providing a feed material (typically a fatty acid or fatty acid derivative), isomerizing a site of unsaturation in the feed material (typically a fatty acid or fatty acid derivative) to produce an isomerized feed material (typically a fatty acid or fatty acid derivative), and then contacting the isomerized material with an alkene in the presence of a metathesis catalyst. The isomerized material can be produced by isomerization with or without subsequent esterification or transesterification. Isomerization can be catalyzed by known biochemical or chemical techniques. For example, an isomerase enzyme, such as a linoleate isomerase, can be used to isomerize linoleic acid from the cis 9, cis 12 isomer to the cis 9, bans 11 isomer. This isomerization process is stereospecific, however, nonstereospecific processes can be used because both cis and trans isomers are suitable for metathesis. For example, an alternative process employs a chemical isomerization catalyst, such as an acidic or basic catalyst, can be used to isomerize an unsaturated feed material (typically a fatty acid or fatty acid derivative) having a site of unsaturation at one location in the molecule into an isomerized, feed material (typically a fatty acid or fatty acid derivative) having a site of unsaturation at a different location in the molecule. Metal or organometallic catalysts also can be used to isomerize an unsaturated feed material (typically a fatty acid or fatty acid derivative). For example, nickel catalysts are known to catalyze positional isomerization of unsaturated sites in fatty acid derivatives. Similarly, esterification, transesterification, reduction, oxidation and/or other modifications of the starting compound or products, such as a fatty acid or fatty acid derivative, can be catalyzed by biochemical or chemical techniques. For example, a fatty acid or fatty acid derivative can be modified by a lipase, esterase, reductase or other enzyme before or after isomerization. In another embodiment the isomerization described above may be practiced with any triacylglycerides, biodiesel, fatty acids, fatty acid esters and/or fatty acid alkyl esters described herein, typically before contacting with the metathesis catalyst.

### Metathesis Catalysts

In a preferred embodiment, the metathesis catalyst is represented by the formula: 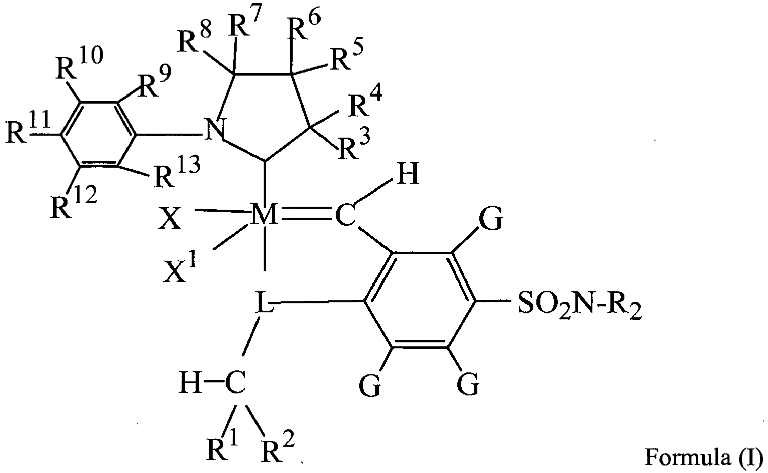 where:
M is a Group 8 metal, preferably Ru or Os, preferably Ru;
X and X¹ are, independently, any anionic ligand, preferably a halogen (preferably C₁), an alkoxide or an alkyl sulfonate, or X and X¹ may be joined to form a dianionic group and may form single ring of up to 30 non-hydrogen atoms or a multinuclear ring system of up to 30 non-hydrogen atoms;
L is O or S, preferably O;
R is hydrogen or a C₁ to C₃₀ hydrocarbyl or substituted hydrocarbyl, preferably methyl;
R¹, R², R³, R⁴, R⁵, R⁶, R⁷, and R⁸ are, independently, hydrogen or a C₁ to C₃₀ hydrocarbyl or substituted hydrocarbyl, preferably methyl, ethyl, propyl or butyl, preferably R¹, R², R³, and R⁴are methyl;
each R⁹ and R¹³ are, independently, hydrogen or a C₁ to C₃₀ hydrocarbyl or substituted hydrocarbyl, preferably a C₂ to C₆ hydrocarbyl, preferably ethyl;
R¹⁰, R¹¹, R¹² are, independently hydrogen or a C₁ to C₃₀ hydrocarbyl or substituted hydrocarbyl, preferably hydrogen or methyl;
each G, is, independently, hydrogen, halogen or C₁ to C₃₀ substituted or unsubstituted hydrocarbyl (preferably a C₁ to C₃₀ substituted or unsubstituted alkyl or a substituted or unsubstituted C₄ to C₃₀ aryl);
where any two adjacent R groups may form a single ring of up to 8 non-hydrogen atoms or a multinuclear ring system of up to 30 non-hydrogen atoms.

Preferably any two adjacent R groups may form a fused ring having from 5 to 8 non hydrogen atoms. Preferably the non-hydrogen atoms are C and or O. Preferably the adjacent R groups form fused rings of 5 to 6 ring atoms, preferably 5 to 6 carbon atoms. By adjacent is meant any two R groups located next to each other, for example R³ and R⁴ can form a ring and/or R¹¹ and R¹² can form a ring.

In a preferred embodiment, at least one of R⁹ and R¹³ is a C₁ to C₃₀ hydrocarbyl or substituted hydrocarbyl, preferably both are C₁ to C₃₀ hydrocarbyl or substituted hydrocarbyl.

For purposes of this invention and claims thereto a substituted hydrocarbyl is a radical made of carbon and hydrogen where at least one hydrogen is replaced by a heteroatom. For purposes of this invention and claims thereto a substituted alkyl or aryl group is a radical made of carbon and hydrogen where at least one hydrogen is replaced by a heteroatom or a linear, branched, or cyclic substituted or unsubstituted hydrocarbyl group having 1 to 30 carbon atoms.

Preferred alkoxides include those where the alkyl group is a phenol, substituted phenol (where the phenol may be substituted with up to 1, 2, 3, 4 or 5 C₁ to C₁₂ hydrocarbyl groups) or a C₁ to C₁₀ hydrocarbyl, preferably a C₁ to C₁₀ alkyl group, preferably methyl, ethyl, propyl, butyl, or phenyl.

Preferred alkyl sulfonates are represented by the Formula (II): 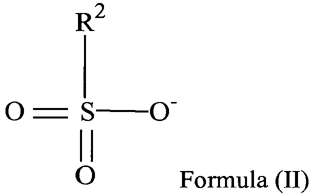 where R² is a C₁ to C₃₀ hydrocarbyl group, fluoro-substituted carbyl group, chloro-substituted carbyl group, aryl group, or substituted aryl group, preferably a C₁ to C₁₂ alkyl or aryl group, preferably trifluoromethyl, methyl, phenyl, para-methyl-phenyl.

Preferred metathesis catalysts include: 2-(2,6-diethylphenyl)-3,3,5,5-tetramethylpyrrolidine[2-(i-propoxy)-5-(N,N-dimethylaminosulfonyl)phenyl]methylene ruthenium dichloride; 2-(mesityl)-3,3,5,5-tetramethylpyrrolidine[2-(i-propoxy)-5-(N,N-dimethylaminosulfonyl)phenyl]methylene ruthenium dichloride; 2-(2-isopropyl)-3,3,5,5-tetramethylpyrrolidine[2-(i-propoxy)-5-(N,N-dimethylaminosulfonyl)phenyl]methylene ruthenium dichloride; 2-(2,6-diethyl-4-fluorophenyl)-3,3,5,5-tetramethylpyrrolidine[2-(i-propoxy)-5-(N,N-dimethylaminosulfonyl)phenyl]methylene ruthenium dichloride, and mixtures thereof.

The catalyst compounds described herein may be synthesized as follows.

The cyclic (alkyl)(amino)carbene precursor, as the aldiminium salt, can be synthesized as reported in the literature (Angew Chem. Int. Ed. 2005, 44, 5705-5709 or in WO 2006/138166). For example 2,6-diethylaniline is reacted with isobutyraldehyde in the presence of 3 angstrom molecular sieves and a catalytic amount of p-toluenesulfonic acid monohydrate at 50°C in benzene. The resulting imine is reacted with a deprotonating agent such as lithium diisopropyl amide which is reacted with 1,2-epoxy-2-methylpropane. Treatment with trifluoromethane sulfonic anhydride yields the aldiminium salt. The aldiminium salt upon deprotonation with the appropriate base such as potassium bistrimethylsilyl amide generates the carbene at low temperatures such as -80°C. This carbene can be reacted with ruthenium alkylidene complexes such as 2-(i-propoxy)-5-(N,N-dimethylaminosulfonyl)phenyl]methylene}(tricyclohexylphosphine) ruthenium dichloride to generate the cyclic alky amino carbene ruthenium complex, 2-(2,6-diethylphenyl)-3,3,5,5-tetramethylpyrrolidine[2-(i-propoxy)-5-(N,N-dimethylaminosulfonyl)phenyl]methylene ruthenium dichloride.

The resulting ruthenium alkylidene complex is an efficient catalyst or catalyst precursor towards for the cometathesis of ethylene and methyl oleate, a component of biodiesel, to generate with good selectivity 1-decene and methyl-9-decenoate. The cometathesis reaction is performed in a suitable solvent such as dichloromethane, toluene, hexane, or other analogous solvents. The reaction is performed at 40 to 50°C, and may be performed at a temperature range of 0°C to 100°C. The ethylene pressure in the reaction vessel is typically in a range of 100 to 200 psi (689,000 to 1,378,000 Pa). It may be in a range of 10 psi to 1000 psi (68,900 to 6,890,000 Pa) of ethylene.

### Alipha-Olefin Products of the Metathesis Reaction.

In a preferred embodiment, the processes described herein produce a linear alpha olefin. The alpha-olefin, preferably linear alpha-olefin, produced herein contains at least one more carbon than the alkene used in the reaction to make the alpha-olefin.

In another embodiment, the processes described herein produce a blend of an alpha olefin and an ester-functionalized alpha olefin. Generally a mixture of non-ester-containing alpha olefins will be produced due to the presence of mono-, di-, and triunsubstituted fatty acid chains. The major alpha olefin products are expected to be 1-decene, 1-heptene, and 1-butene. The major ester-containing alpha olefin product is methyl dec-9-enoate.

In a preferred embodiment, the alpha olefin produced herein is 1-decene. Typically the 1-decene is produced with an ester.

In a preferred embodiment, the major alpha olefin produced herein is 1-decene. Typically the 1-decene is produced with an ester.

In a preferred embodiment, ethylene and methyl oleate are combined with the metathesis catalysts described herein (such as 2-(2,6-diethylphenyl)-3,3,5,5-tetramethylpyrrolidine[2-(i-propoxy)-5-(N,N-dimethylaminosulfonyl)phenyl]methylene ruthenium dichloride) to produce 1-decene and methyl dec-9-enoate.

Separation of the 1-olefin (such as the 1-decene) from the ester may be by means typically know in the art such as distillation or filtration.

The linear alpha-olefin (such as 1-decene or a mixture of C₈, C₁₀, C₁₂ linear alpha olefins) is then separated from any esters present and preferably used to make poly-alpha-olefins(PAOs). Specifically, PAOs may be produced by the polymerization of olefin feed in the presence of a catalyst such as AlCl₃, BF₃, or BF₃ complexes. Processes for the production of PAOs are disclosed, for example, in the following patents: U.S. Patents 3,149,178; 3,382,291; 3,742,082; 3,769,363; 3,780,128; 4,172,855; and 4,956,122; which are fully incorporated by reference. PAOs are also discussed in: Will, J.G. Lubrication Fundamentals, Marcel Dekker: New York, 1980. Certain high viscosity index PAO's may also be conveniently made by the polymerization of an alpha-olefin in the presence of a polymerization catalyst such as Friedel-Crafts catalysts. These include, for example, aluminum trichloride, boron trifluoride, aluminum trichloride or boron trifluoride promoted with water, with alcohols such as ethanol, propanol, or butanol, with carboxylic acids, or with esters such as ethyl acetate or ethyl propionate or ether such as diethyl ether, diisopropyl ether, etc., see for example, the methods disclosed by U.S. Patents 4,149,178; 3,382,291; 3,742,082; 3,769,363 (Brennan); 3,876,720; 4,239,930; 4,367,352; 4,413,156; 4,434,408; 4,910,355; 4,956,122; 5,068,487; 4,827,073; 4,827,064; 4,967,032; 4,926,004; and 4,914,254. PAO's can also be made using various metallocene catalyst systems. Examples include US 6,706,828 WO 96/23751, EP 0 613 873, US 5,688,887, US 6,043,401, WO 03/020856, US 6,548,724, US 5,087,788, US 6,414,090, US 6,414,091, US 4,704,491, US 6,133,209, and US 6,713,438.

PAOs are often used as additives and base stocks for lubricants, among other things. Additional information on the use of PAO's in the formulations of full synthetic, semi-synthetic or part synthetic lubricant or functional fluids can be found in "Synthetic Lubricants and High-Performance Functional Fluids", 2nd Ed. L. Rudnick, etc. Marcel Dekker, Inc., N.Y. (1999). Additional information on additives used in product formulation can be found in "Lubricants and Lubrications, Ed. By T. Mang and W. Dresel, by Wiley-VCH GmbH, Weinheim 2001.

In another embodiment this disclosure relates to:
1. A metathesis catalyst compound represented by the formula: 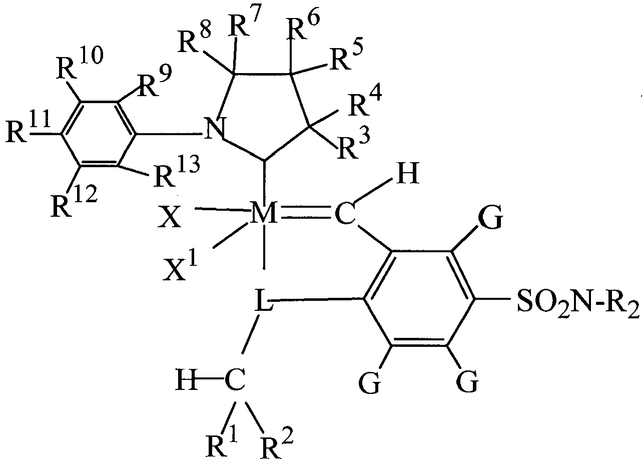 where:
   M is a Group 8 metal;
   X and X¹ are, independently, any anionic ligand, or X and X¹ may be joined to form a dianionic group and may form single ring of up to 30 non-hydrogen atoms or a multinuclear ring system of up to 30 non-hydrogen atoms;
   L is O or S;
   R is hydrogen or a C₁ to C₃₀ hydrocarbyl or substituted hydrocarbyl;
   R¹, R², R³, R⁴, R⁵, R⁶, R⁷, and R⁸ are, independently, hydrogen or a C₁ to C₃₀ hydrocarbyl or substituted hydrocarbyl;
   each R⁹ and R¹³ are, independently, hydrogen, a C₁ to C₃₀ hydrocarbyl or substituted hydrocarbyl;
   R¹⁰, R¹¹, R¹² are, independently, hydrogen or a C₁ to C₃₀ hydrocarbyl or substituted hydrocarbyl;
   each G, is, independently, hydrogen, halogen or C₁ to C₃₀ substituted or unsubstituted hydrocarbyl;
   where any two adjacent R groups may form a single ring of up to 8 non-hydrogen atoms or a multinuclear ring system of up to 30 non-hydrogen atoms.
2. The catalyst compound of paragraph 1, wherein M is ruthenium.
3. The catalyst compound of paragraph 1 or 2, wherein X and X¹ are, independently, a halogen, an alkoxide or an alkyl sulfonate.
4. The catalyst compound of any of paragraphs 1 to 3, wherein X and X¹ are Cl.
5. The catalyst compound of any of paragraphs 1 to 4, wherein L is N or O.
6. The catalyst compound of any of paragraphs 1 to 5, wherein R is a C₁ to C₃₀ hydrocarbyl.
7. The catalyst compound of any of paragraphs 1 to 6, wherein R is methyl.
8. The catalyst compound of any of paragraphs 1 to 7, wherein each G is independently, a C₁ to C₃₀ substituted or unsubstituted alkyl, or a substituted or unsubstituted C₄ to C₃₀ aryl.
9. The catalyst compound of any of paragraphs 1 to 8, wherein L is N.
10. The catalyst compound of paragraph 1, wherein the metathesis catalyst compound comprises one or more of: 2-(2,6-diethylphenyl)-3,3,5,5-tetramethylpyrrolidine[2-(i-propoxy)-5-(N,N-dimethylaminosulfonyl)phenyl]methylene ruthenium dichloride; 2-(mesityl)-3,3,5,5-tetramethylpyrrolidine[2-(i-propoxy)-5-(N,N-dimethylaminosulfonyl)phenyl]methylene ruthenium dichloride; 2-(2-isopropyl)-3,3,5,5-tetramethylpyrrolidine[2-(i-propoxy)-5-(N,N-dimethylaminosulfonyl)phenyl]methylene ruthenium dichloride; 2-(2,6-diethyl-4-fluorophenyl)-3,3,5,5-tetramethylpyrrolidine[2-(i-propoxy)-5-(N,N-dimethylaminosulfonyl)phenyl]methylene ruthenium dichloride, or mixtures thereof.
11. A process to produce alpha-olefin comprising contacting a seed oil with the catalyst compound of any of paragraphs 1 to 10.
12. The process of paragraph 11, wherein the seed oil is selected from the group consisting of canola oil, corn oil, soybean oil, rapeseed oil, algae oil, peanut oil, mustard oil, sunflower oil, tung oil, perilla oil, grapeseed oil, linseed oil, safflower oil, pumpkin oil, palm oil, Jathropa oil, high-oleic soybean oil, high-oleic safflower oil, high-oleic sunflower oil, mixtures of animal and vegetable fats and oils, castor bean oil, dehydrated castor bean oil, cucumber oil, poppyseed oil, flaxseed oil, lesquerella oil, walnut oil, cottonseed oil, meadowfoam, tuna oil, sesame oils and mixtures thereof.
13. The process of paragraph 11, wherein the seed oil is selected from the group consisting of palm oil and algae oil.
14. A process to produce alpha-olefin comprising contacting a triacylglyceride with an alkene and the catalyst compound of any of paragraphs 1 to 10, preferably wherein the alpha olefin produced has at least one more carbon atom than the alkene.
15. The process of paragraph 14, wherein the triacylglyceride is contacted with alcohol and converted to an fatty acid ester or fatty acid alkyl ester prior to contacting with the catalyst compound of any of paragraphs 1 to 10.
16. The process of paragraph 14, wherein the triacylglyceride is contacted with water or an alkaline reagent and converted to a fatty acid prior to contacting with the catalyst compound of any of paragraphs 1 to 10.
17. A process to produce alpha-olefin comprising contacting an unsaturated fatty acid with an alkene and the catalyst compound of any of paragraphs 1 to 10, preferably wherein the alpha olefin produced has at least one more carbon atom than the alkene.
18. A process to produce alpha-olefin comprising contacting a triacylglyceride with the catalyst compound of any of paragraphs 1 to 10, preferably wherein the alpha olefin produced has at least one more carbon atom than the alkene.
19. A process to produce alpha-olefin comprising contacting an unsaturated fatty acid ester and or unsaturated fatty acid alkyl ester with an alkene and the catalyst compound of any of paragraphs 1 to 10, preferably wherein the alpha olefin produced has at least one more carbon atom than the alkene.
20. The process of any of paragraphs 11 to 19, wherein the alpha olefin is a linear alpha-olefin having 4 to 24 carbon atoms.
21. The process of any of paragraphs 11 to 20, wherein the alkene is ethylene, propylene, butene, hexene or octene.
22. The process of any of paragraphs 19 to 21, wherein the fatty acid ester is a fatty acid methyl ester.
23. The process of any of paragraphs 14 to 22, wherein the triacylglyceride, fatty acid, fatty acid alkyl ester, fatty acid ester is derived from biodiesel.
24. The process of any of paragraphs 11 to 23, wherein the alpha-olefin is butene-1, decene-1 and or heptene-1.
25. The process of any of paragraphs 11 to 24, wherein the productivity of the process is at least 200 g of linear alpha-olefin per mmol of catalyst per hour.
26. The process of any of paragraphs 11 to 25, wherein the selectivity of the process is at least 20 wt% linear alpha-olefin, based upon the weight to the material exiting the reactor.
27. The process of any of paragraphs 11 to 26, wherein the turnover number, defined as the moles of alpha olefin formed per mol of catalyst, of the process is at least 10,000.
28. The process of any of paragraphs 11 to 27, wherein the yield, when converting unsaturated fatty acids, unsaturated fatty acid esters, unsaturated fatty acid alkyl esters or mixtures thereof, is 30% or more, said yield being defined as defined as the moles of alpha olefin formed per mol of unsaturated fatty acids, unsaturated fatty acid esters, unsaturated fatty acid alkyl esters or mixtures thereof introduced into the reactor.
29. The process of any of paragraphs 11 to 27, wherein the yield, when converting TAGs as represented in the formula below, is 30% or more, said yield being defined as defined as the moles of alpha olefin formed divided by (the moles of unsaturated R^{a} + moles of unsaturated R^{b} + moles of unsaturated R^{c}) introduced into the reactor, 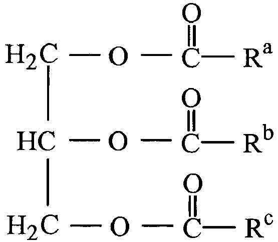 where R^{a}, R^{b} and R^{c} each, independently, represent a saturated or unsaturated hydrocarbon chain.
30. The process of paragraph 28, wherein the yield is 60% or more.
31. A process to produce C₄ to C₂₄ linear alpha-olefin comprising contacting a feed material with an alkene selected from the group consisting of ethylene, propylene butene, pentene, hexene, heptene, octene, nonene and mixtures thereof and a metathesis catalyst compound of any of paragraphs 1 to 10, wherein the feed material is a triacylglyceride, fatty acid, fatty acid alkyl ester, and/or fatty acid ester derived from seed oil.
32. The process of paragraph 31, wherein the alkene is ethylene, the alpha olefin is 1-butene, 1-heptene and or -decene, and the feed material is a fatty acid methyl ester, and/or fatty acid ester.

### EXPERIMENTAL SECTION

### Tests and Materials

All molecular weights are number average unless otherwise noted. All molecular weights are reported in g/mol unless otherwise noted.

THF is tetrahydrofuran.

Products were analyzed by gas chromatography (Agilent 6890N with auto-injector) using helium as a carrier gas at 38 cm/sec. A column having a length of 60 m(J & W Scientific DB-1, 60 m x 0.25 mm I.D.x 1.0 µm film thickness) packed with a flame ionization detector (FID), an Injector temperature of 250°C, and a Detector temperature of 250°C were used. The sample injected into the column in an oven at 70°C, then heated to 275°C over 22 minutes (ramp rate 10°C/min to 100°C, 30°C/min to 275°C, hold).

### EXAMPLES

### Synthesis Example 1

**Synthesis of (E)-2,6-diethyl-N-(2-methylpropylidene)aniline (compound 1).** Benzene (150 mL) was added to 2,6-diethylaniline (18.59 g, 124.6 mmol) and 3 angstrom molecular sieves (ca. 50 mL). Then isobutyraldehyde (9.43 g, 131 mmol) and p-toluenesulfonic acid monohydrate (20 mg, 0.011 mmol) were added. The flask was sealed and heated to 50°C. After stirring overnight the very pale yellow solution was filtered and the volatiles were removed under reduced pressure to afford the product as a clear, pale yellow oil. Yield: 22.5 g, 84.6%. ¹H NMR (C₆D₆): δ 7.21 (1H, d), 7.02 (2H, m), 2.47 (4H, q), 2.39 (1H, m), 1.11 (6H, t), 1.01 (6H, d).

**Synthesis of lithium (2,6-diethylphenyl)(2-methylprop-1-enyl)amide (compound 2).** Benzene (70 mL) and compound 1 (6.63 g, 32.6 mmol) were combined. Then solid lithium diisopropylamide (4.01 g, 37.4 mmol) was added. The mixture was heated to 50°C to form a cloudy red-orange solution. After a few hours the solution was filtered through diatomaceous earth to afford a clear yellow solution. The volatiles were evaporated to give a yellow solid. Pentane (15 mL) was added and the mixture was stirred briefly and then cooled to -10°C overnight. The product was then collected on a glass frit and washed with pentane (2 x 20 mL) to give a white solid that was dried under reduced pressure. Yield: 5.50 g, 80.6%.

**Synthesis of 1-(2,6-diethylphenyl)-2,2,4,4-tetramethyl-3,4-dihydro-2H-pyrrolium triflate (compound 3).** Et₂O (100 mL) was added to compound 2 (5.50 g, 26.3 mmol) to form a clear yellow solution. An Et₂O (5 mL) solution of 1,2-epoxy-2-methylpropane (1.90 g, 26.3 mmol) was added dropwise over about 10 seconds, and the mixture was stirred overnight. The next day the mixture was cooled to -80°C and trifluoromethanesulfonic anhydride (7.42 g, 26.3 mmol) was added dropwise. The mixture was warmed to ambient temperature over 1 hour. A thick suspension formed. After stirring for an additional hour, the solid was collected on a glass frit and washed with Et₂O (3 x 15 mL). The solid was dried under reduced pressure. This was then extracted with CH₂Cl₂ (60 mL) and filtered through diatomaceous earth. The filter cake was washed with CH₂Cl₂ (2 x 30 mL). The combined CH₂Cl₂ extracts were evaporated to an oil and Et₂O (15 mL) was added to cause a white crystalline solid to form. The solution was cooled to -10°C overnight. The solid was then collected and dried under reduced pressure to afford 1.48 g of product. To obtain additional product the filter cake was loaded into a thimble. This was placed in a Soxhlet extractor and the solid was extracted with hot CH₂Cl₂ overnight. The resulting CH₂Cl₂ extract was evaporated and Et₂O (10 mL) was added to afford additional product as off-white crystals. Total yield: 3.28 g, 30.6%. ¹H NMR (C₆D₆): δ 7.21 (1H, d), 7.02 (2H, m), 2.47 (4H, q), 2.39 (1H, m), 1.11 (6H, t), 1.01 (6H, d).

**Synthesis of 2-(2,6-diethylphenyl)-3,3,5,5-tetramethylpyrrolidine[2-(i-propoxy)-5-(N,N-dimethylaminosulfonyl)phenyl]methylene ruthenium dichloride (compound 4).** Tetrahydrofuran (40 mL) was added to KN(SiMe3)2 (0.379 g, 1.90 mmol) to form a homogeneous solution. At -80°C solution of compound 3 (0.775 g, 1.90 mmol) and THF (10 mL) was added dropwise over about 10 seconds. After 30 seconds a THF (10 mL) solution of {[2-(i-propoxy)-5-(N,N-dimethyl-aminosulfonyl)phenyl]methylene}-(tricyclohexylphosphine) ruthenium dichloride (0.612 g, 0.865 mmol), which had been cooled to -10°C, was added dropwise over about 15 seconds. The mixture was stirred for 10 minutes then warmed to ambient temperature. The mixture was then stirred for 2 h, then the volatiles were removed under reduced pressure. The residue was extracted with 20 mL of a 3:2 mixture of hexane:CH₂Cl₂ and filtered. This solution was loaded on to a SiO₂ column (1.25" x 8") that had been packed with the same solvent mixture. The column was eluted with 3:2 hexane:CH₂Cl₂ (300 mL) and then the solvent strength was gradually increased to pure CH₂Cl₂. The product eluted as a dark green band. Removal of the volatiles afforded a dark green oil that crystallized upon the addition of pentane (2 mL) and cooling to -10°C overnight. The product was isolated as green flocculent crystals that were dried under reduced pressure (0.038 g, 6.4%). ¹H NMR (CD₂Cl₂): δ 16.26 (1H, s, RuC*H*-), 7.96 (1H, dd), 7.64 (1H, t), 7.47 (2H, d), 7.18 (1H, d, J = 2 Hz), 7.11 (1H, d), 5.22 (1H, sept), 2.64 (6H, s), 2.53 (4H, m), 2.19 (2H, s), 2.07 (6H, s), 1.77 (6H, d), 1.32 (6H, s), 0.88 (6H, t).

### Cross Metathesis of Methyl Oleate with Ethylene.

### Example 1

A stock solution was made by dissolving 4.5 mg of **2-(2,6-diethylphenyl)-3,3,5,5-tetramethylpyrrolidine[2-(i-propoxy)-5-(N,N-dimethylaminosulfonyl) phenyl]methylene ruthenium dichloride** in 25 mL anhydrous dichloromethane. 1.0 mL (0.87 g) methyl oleate, 1.26 g (125 nmol catalyst) of catalyst stock solution, 3.7 g anhydrous dichloromethane, and 0.152 g (0.2 mL) tetradecane as an internal standard were weighed out and then placed in a Fisher-Porter bottle equipped with a stir bar. The vessel was then filled with ethylene to 150 psig (1034 kPa) and placed in an oil bath heated to 40°C for 2 hours. After completion the vessel was depressurized and approximately 0.5 mL ethyl vinyl ether was added to stop the reaction. A sample was then taken and analyzed by GC as described above.

### Example 2

This example was run according to the procedure in Example 1, except that the amount of catalyst for this run was halved such that 0.63 g (62.5 nmol catalyst) of catalyst stock solution was added along with 4.4 g anhydrous dichloromethane. The amount of methyl oleate (1.0 mL, 0.876 g) and tetradecane standard (0.2 mL, 0.152 g) remained the same. The reactants were placed in a Fisher-Porter bottle and into a 40°C oil bath for 2 hours, after which ethyl vinyl ether was added to stop the reaction. A sample was taken and analyzed by GC as described above.

### Example 3

This example was run according to the procedure in Example 2, except that the amount of catalyst was for this run was halved from the previous run to 0.31 g (31.25 nmol catalyst) of catalyst stock solution which was added along with 4.7 g anhydrous dichloromethane. The amount of methyl oleate and internal standard remained the same. The reactants were placed in a Fisher-Porter bottle and into a 40°C oil bath for 2 hours, after which ethyl vinyl ether was added to stop the reaction. A sample was taken and analyzed by GC as described above.

### Example 4

This example was run according to the procedure in Example 3, except that the catalyst loading was halved once more to 0.157 g (15.62 nmol catalyst) of catalyst stock solution along with 4.9 g anhydrous dichloromethane. Methyl oleate and standard amounts remained the same. The reactants were placed in a Fisher-Porter bottle and into a 40°C oil bath for 2 hours, after which ethyl vinyl ether was added to stop the reaction. A sample was taken and analyzed by GC as described above.

### Example 5

This example was run according to the procedure in Example 4, expect that the catalyst loading was increased to 0.235 g (23.43 nmol catalyst) of stock solution with 4.8 g anhydrous dichloromethane. Methyl oleate and standard amounts remained the same. The reactants were placed in a Fisher-Porter bottle and into a 40°C oil bath for 2 hours, after which ethyl vinyl ether was added to stop the reaction. A sample was taken and analyzed by GC as described above.

In Table 2, % yield is shown as a percentage and defined as 100×[micromoles of 1-decene]/[micromoles of methyl oleate weighed into reactor]. 1-decene selectivity is shown as a percentage and is defined as 100×[peak area of 1-decene and methyl-9-decenoate]/[sum of peak areas of 1-decene, methyl-9-decenoate, and the homometathesis products, 9-octadecene, and 1,18-dimethyl-9-octadecenedioate]. Catalyst turnover for production of the 1-decene is defined as the [micromoles of 1-decene]/([micromoles of catalyst].

**Table 2. Reaction conditions, 40°C, 2 hours**

| Example | nmoles Catalyst | % Selectivity | % Yield | No. Turnovers 1-Decene |
|---|---|---|---|---|
| 1 | 125 | 92.0 | 62.8 | 14,700 |
| 2 | 62.5 | 88.2 | 52.9 | 24,800 |
| 3 | 31.25 | 90.2 | 46.7 | 43,840 |
| 5 | 23.43 | 92.4 | 43.6 | 52,400 |
| 4 | 15.62 | 91.0 | 31.7 | 59.400 |

## Claims

1. A metathesis catalyst compound represented by the formula: 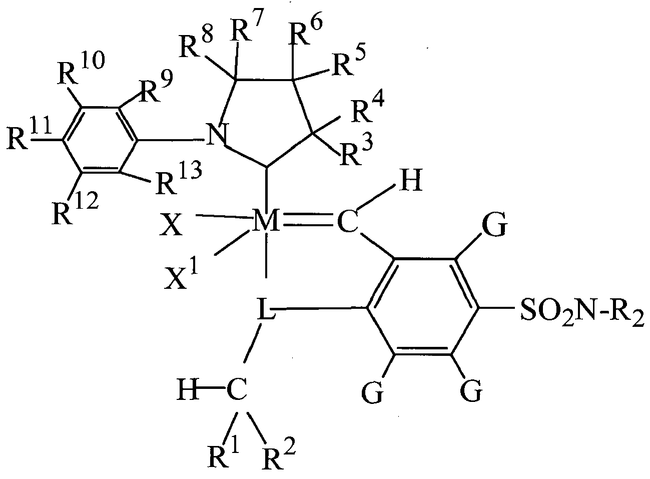 where:
M is a Group 8 metal, preferably Ru;
X and X¹ are, independently, any anionic ligand, or X and X¹ may be joined to form a dianionic group and may form single ring of up to 30 non-hydrogen atoms or a multinuclear ring system of up to 30 non-hydrogen atoms;
L is O or S;
R is hydrogen or a C₁ to C₃₀ hydrocarbyl or substituted hydrocarbyl;
R¹, R², R³, R⁴, R⁵, R⁶, R⁷, and R⁸ are, independently, hydrogen or a C₁ to C₃₀ hydrocarbyl or substituted hydrocarbyl;
each R⁹ and R¹³ are, independently, hydrogen, a C₁ to C₃₀ hydrocarbyl or substituted hydrocarbyl;
R¹⁰, R¹¹, R¹² are, independently, hydrogen or a C₁ to C₃₀ hydrocarbyl or substituted hydrocarbyl;
each G, is, independently, hydrogen, halogen or C₁ to C₃₀ substituted or unsubstituted hydrocarbyl;
where any two adjacent R groups may form a single ring of up to 8 non-hydrogen atoms or a multinuclear ring system of up to 30 non-hydrogen atoms.

2. The catalyst compound of claim 1, wherein X and X¹ are, independently, a halogen, an alkoxide, or an alkyl sulfonate; L is O; R is a C₁ to C₃₀ hydrocarbyl; each G is independently, a C₁ to C₃₀ substituted or unsubstituted alkyl, or a substituted or unsubstituted C₄ to C₃₀ aryl.

3. The catalyst compound of claim 1 wherein the metathesis catalyst compound comprises one or more of: 2-(2,6-diethylphenyl)-3,3,5,5-tetramethylpyrrolidine[2-(i-propoxy)-5-(N,N-dimethylaminosulfonyl)phenyl]methylene ruthenium dichloride; 2-(mesityl)-3,3,5,5-tetramethylpyrrolidine[2-(i-propoxy)-5-(N,N-dimethylaminosulfonyl)phenyl]methylene ruthenium dichloride; 2-(2-isopropyl)-3,3,5,5-tetramethylpyrrolidine[2-(i-propoxy)-5-(N,N-dimethylaminosulfonyl)phenyl]methylene ruthenium dichloride; 2-(2,6-diethyl-4-fluorophenyl)-3,3,5,5-tetramethylpyrrolidine[2-(i-propoxy)-5-(N,N-dimethylaminosulfonyl)phenyl]methylene ruthenium dichloride, or mixtures thereof.

4. A process to produce alpha-olefin comprising contacting a seed oil with the catalyst compound of any of claims 1 to 3, wherein the alpha olefin is preferably a linear alpha-olefin having 4 to 24 carbon atoms and is more preferably butene-1, decene-1 and or heptene-1.

5. The process of claim 4, wherein the seed oil is selected from the group consisting of canola oil, corn oil, soybean oil, rapeseed oil, algae oil, peanut oil, mustard oil, sunflower oil, tung oil, perilla oil, grapeseed oil, linseed oil, safflower oil, pumpkin oil, palm oil, Jathropa oil, high-oleic soybean oil, high-oleic safflower oil, high-oleic sunflower oil, mixtures of animal and vegetable fats and oils, castor bean oil, dehydrated castor bean oil, cucumber oil, poppyseed oil, flaxseed oil, lesquerella oil, walnut oil, cottonseed oil, meadowfoam oil, tuna oil, sesame oils and mixtures thereof.

6. The process of claim 4 comprising contacting a triacylglyceride with an alkene and the catalyst compound of any of claims 1 to 3, preferably wherein the alpha olefin produced has at least one more carbon atom than the alkene.

7. The process of claim 6, wherein the triacylglyceride is contacted with alcohol and converted to an fatty acid ester or fatty acid alkyl ester prior to contacting with the alkene and the catalyst compound of any of claims 1 to 3, or wherein the triacylglyceride is contacted with water or an alkaline reagent and converted to a fatty acid prior to contacting with the alkene and the catalyst compound of any of claims 1 to 3.

8. The process of claim 4 comprising contacting an unsaturated fatty acid or unsaturated fatty acid ester, preferably unsaturated fatty acid alkyl ester, such as a fatty acid methyl ester, with an alkene and the catalyst compound of any of claims 1 to 3, preferably wherein the alpha olefin produced has at least one more carbon atom than the alkene.

9. The process of any of claims 6 to 8, wherein the alkene is ethylene, propylene, butene, hexene, or octene.

10. The process of any of claims 7 to 9, wherein the triacylglyceride, fatty acid, fatty acid alkyl ester, fatty acid ester is derived from biodiesel.

11. The process of any of claims 4 to 10, wherein the process has one or more of the following characteristics:
- the productivity of the process is at least 200 g of linear alpha-olefin per mmol of catalyst per hour;
- the selectivity of the process is at least 20 wt% linear alpha-olefin, based upon the weight to the material exiting the reactor;
- the turnover number, defined as the moles of alpha olefin formed per mol of catalyst, of the process is at least 10,000;
- the yield, when converting unsaturated fatty acids, unsaturated fatty acid esters, unsaturated fatty acid alkyl esters or mixtures thereof, is 30% or more, preferably 60% or more, said yield being defined as defined as the moles of alpha olefin formed per mol of unsaturated fatty acids, unsaturated fatty acid esters, unsaturated fatty acid alkyl esters or mixtures thereof introduced into the reactor;
- the yield, when converting TAGs as represented in the formula below, is 30% or more, said yield being defined as defined as the moles of alpha olefin formed divided by (the moles of unsaturated R^{a} + moles of unsaturated R^{b} + moles of unsaturated R^{c}) introduced into the reactor, 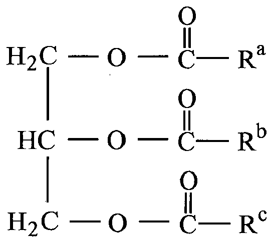
where R^{a}, R^{b} and R^{c} each, independently, represent a saturated or unsaturated hydrocarbon chain.

12. The process of any of claims 4 to 11 to produce C₄ to C₂₄ linear alpha-olefin comprising contacting a feed material with an alkene selected from the group consisting of ethylene, propylene butene, pentene, hexene, heptene, octene, nonene and mixtures thereof and a metathesis catalyst compound of any of claims 1 to 3, wherein the feed material is a triacylglyceride, fatty acid, fatty acid alkyl ester, and/or fatty acid ester derived from seed oil.

13. The process of claim 12, wherein the alkene is ethylene, the alpha olefin is 1-butene, 1-heptene and or -decene, and the feed material is a fatty acid methyl ester, and/or fatty acid ester.

## Patentansprüche

1. Metathesekatalysatorverbindung mit der Formel: 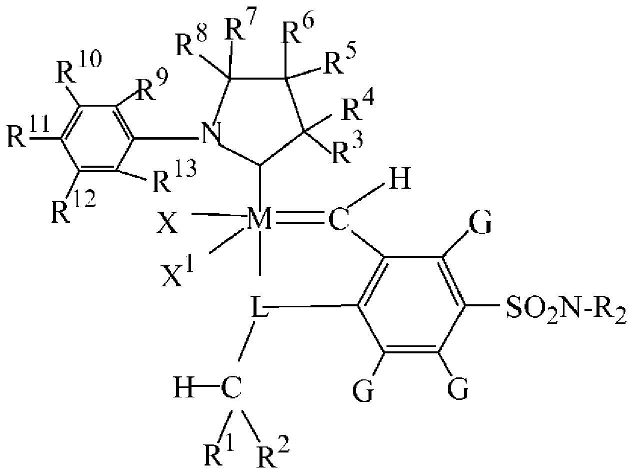 wobei:
M ein Metall der Gruppe 8, vorzugsweise Ru ist;
X und X¹ unabhängig jeweils ein beliebiger anionischer Ligand sind, oder X und X¹ unter Bildung einer dianionischen Gruppe verbunden sein können und einen einzelnen Ring mit bis zu 30 Nicht-Wasserstoffatomen oder ein mehrkerniges Ringsystem mit bis zu 30 Nicht-Wasserstoffatomen bilden können;
L O oder S ist;
R Wasserstoff oder ein C₁- bis C₃₀-Kohlenwasserstoffrest oder substituierter Kohlenwasserstoffrest ist;
R¹, R², R³, R⁴, R⁵, R⁶, R⁷ und R⁸ unabhängig Wasserstoff oder ein C₁- bis C₃₀-Kohlenwasserstoffrest oder substituierter Kohlenwasserstoffrest sind;
jedes R⁹ und R¹³ unabhängig Wasserstoff, ein C₁- bis C₃₀-Kohlenwasserstoffrest oder substituierter Kohlenwasserstoffrest ist;
R¹⁰, R¹¹, R¹² unabhängig Wasserstoff oder ein C₁- bis C₃₀-Kohlenwasserstoffrest oder substituierter Kohlenwasserstoffrest sind;
jedes G unabhängig Wasserstoff, Halogen oder substituierter oder unsubstituierter C₁- bis C₃₀-Kohlenwasserstoff-rest ist;
wobei beliebige zwei benachbarten R-Gruppen einen einzelnen Ring mit bis zu 8 Nicht-Wasserstoffatomen oder ein mehrkerniges Ringsystem mit bis zu 30 Nicht-Wasserstoffatomen bilden können.

2. Katalysatorverbindung nach Anspruch 1, bei der X und X¹ unabhängig ein Halogen, ein Alkoxid oder ein Alkylsulfonat sind; L O ist; R ein C₁- bis C₃₀-Kohlenwasserstoffrest ist; jedes G unabhängig ein substituiertes oder unsubstituiertes C₁- bis C₃₀-Alkyl oder ein substituiertes oder unsubstituiertes C₄- bis C₃₀-Aryl ist.

3. Katalysatorverbindung nach Anspruch 1, bei der die Metathesekatalysatorverbindung ein oder mehrere von 2-(2,6-Diethylphenyl)-3,3,5,5-tetramethylpyrrolidin[2-(i-prop-oxy)-5-(N,N-dimethylaminosulfonyl)phenyl]methylenrutheni-umdichlorid; 2-(Mesityl)-3,3,5,5-tetramethylpyrrolidin[2-(i-propoxy)-5-(N,N-dimethylaminosulfonyl)phenyl]methylen-rutheniumdichlorid; 2-(2-Isopropyl)-3,3,5,5-tetramethyl-pyrrolidin[2-(i-propoxy)-5-(N,N-dimethylaminosulfonyl)-phenyl]methylenrutheniumdichlorid ; 2-(2,6-Diethyl-4-fluorphenyl)-3,3,5,5-tetramethylpyrrolidin[2-(i-propoxy)-5-(N,N-dimethylaminosulfonyl)phenyl]methylenrutheniumdi-chlorid oder Mischungen davon umfasst.

4. Verfahren zur Herstellung von α-Olefin, bei dem ein Samenöl mit der Katalysatorverbindung gemäß einem der Ansprüche 1 bis 3 kontaktiert wird, wobei das α-Olefin vorzugsweise ein lineares α-Olefin mit 4 bis 24 Kohlenstoffatomen und insbesondere Buten-1, Decen-1 und/oder Hepten-1 ist.

5. Verfahren nach Anspruch 4, bei dem das Samenöl ausgewählt ist aus der Gruppe bestehend aus Canolaöl, Maisöl, Sojaöl, Rapsöl, Algenöl, Erdnussöl, Senföl, Sonnenblumenöl, Tungöl, Perillaöl, Traubenkernöl, Leinsamenöl, Distelöl, Kürbisöl, Palmöl, Jathropaöl, ölsäurereichem Sojaöl, ölsäurereichem Distelöl, ölsäurereichem Sonnenblumenöl, Mischungen von tierischen und pflanzlichen Fetten und Ölen, Rizinusöl, dehydratisiertem Rizinusöl, Gurkenöl, Mohnsamenöl, Flachssamenöl, Lesquerellaöl, Walnussöl, Baumwollsamenöl, Sumpfblumenöl (Wiesenschaumkrautöl; Meadowfoam-Öl), Thunfischöl, Sesamölen und Mischungen davon.

6. Verfahren nach Anspruch 4, bei dem ein Triacylglycerid mit einem Alken und der Katalysatorverbindung gemäß einem der Ansprüche 1 bis 3 kontaktiert wird, wobei vorzugsweise das produzierte α-Olefin mindestens ein Kohlenstoffatom mehr als das Alken aufweist.

7. Verfahren nach Anspruch 6, bei dem das Triacylglycerid mit Alkohol kontaktiert wird und in einen Fettsäureester oder Fettsäurealkylester umgewandelt wird, bevor das Kontaktieren mit dem Alken und der Katalysatorverbindung gemäß einem der Ansprüche 1 bis 3 erfolgt, oder wobei das Triacylglycerid mit Wasser oder einem alkalischen Reagenz kontaktiert und in eine Fettsäure umgewandelt wird, bevor das Kontaktieren mit dem Alken und der Katalysatorverbindung gemäß einem der Ansprüche 1 bis 3 erfolgt.

8. Verfahren nach Anspruch 4, bei dem eine ungesättigte Fettsäure oder ein ungesättigter Fettsäureester, vorzugsweise ungesättigter Fettsäurealkylester, wie Fettsäuremethylester, mit einem Alken und der Katalysatorverbindung gemäß einem der Ansprüche 1 bis 3 kontaktiert wird, wobei das produzierte α-Olefin vorzugsweise mindestens ein Kohlenstoffatom mehr als das Alken aufweist.

9. Verfahren nach einem der Ansprüche 6 bis 8, bei dem das Alken Ethylen, Propylen, Buten, Hexen oder Octen ist.

10. Verfahren nach einem der Ansprüche 7 bis 9, bei dem das Triacylglycerid, die Fettsäure, der Fettsäurealkylester, der Fettsäureester von Biodiesel abgeleitet ist.

11. Verfahren nach einem der Ansprüche 4 bis 10, das ein oder mehrere der folgenden Charakteristika aufweist:
- die Produktivität des Verfahrens beträgt mindestens 200 g lineares α-Olefin pro mmol Katalysator pro Stunde;
- die Selektivität des Verfahrens beträgt mindestens 20 Gew.% lineares α-Olefin, bezogen auf das Gewicht des Materials, das den Reaktor verlässt;
- die Umsatzzahl, definiert als die Mol α-Olefin, die pro Mol Katalysator gebildet werden, des Verfahrens beträgt mindestens 10 000;
- die Ausbeute beim Umwandeln von ungesättigten Fettsäuren, ungesättigten Fettsäureestern, ungesättigten Fettsäurealkylestern oder Mischungen davon beträgt 30 % oder mehr, vorzugsweise 60 % oder mehr, wobei die Ausbeute als Mol α-Olefin definiert ist, die pro Mol ungesättigten Fettsäuren, ungesättigten Fettsäureestern, ungesättigten Fettsäurealkylestern oder Mischungen davon gebildet werden, die in den Reaktor eingebracht werden;
- die Ausbeute bei der Umwandlung von TAGs, die in der folgenden Formel wiedergegeben werden, beträgt 30 % oder mehr, wobei die Ausbeute definiert ist als die Mol von gebildetem α-Olefin, geteilt durch (die Mol an ungesättigtem R^{a} + Mol an ungesättigtem R^{b} + Mol an ungesättigtem R^{c}), die in den Reaktor eingebracht werden, 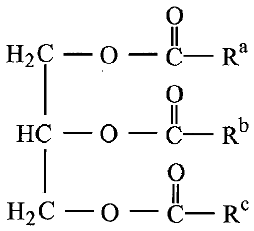
wobei R^{a}, R^{b} und R^{c} jeweils für eine gesättigte oder ungesättigte Kohlenwasserstoffkette stehen.

12. Verfahren nach einem der Ansprüche 4 bis 11 zur Herstellung von linearem C₄- bis C₂₄-α-Olefin, bei dem ein Einsatzmaterial mit einem Alken ausgewählt aus der Gruppe bestehend aus Ethylen, Propylen, Buten, Penten, Hexen, Hepten, Octen, Nonen und Mischungen davon und einer Metathesekatalysatorverbindung gemäß einem der Ansprüche 1 bis 3 kontaktiert wird, wobei das Einsatzmaterial ein Triacylglycerid, eine Fettsäure, ein Fettsäurealkylester und/oder ein Fettsäureester ist, der von Samenöl abgeleitet ist.

13. Verfahren nach Anspruch 12, bei dem das Alken Ethylen ist, das α-Olefin 1-Buten, 1-Hepten oder 1-Decen ist, und das Einsatzmaterial ein Fettsäuremethylester und/oder Fettsäureester ist.

## Revendications

1. Composé catalyseur de métathèse représenté par la formule : 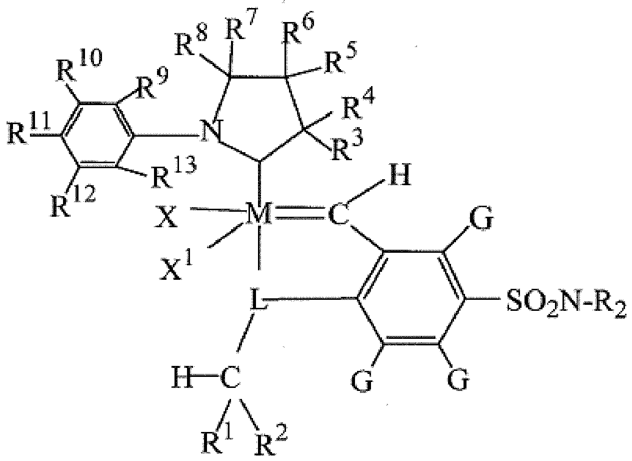 dans laquelle :
M représente un métal du Groupe 8, de préférence Ru ;
X et X¹ représentent, indépendamment, un quelconque ligand anionique, ou bien X et X¹ peuvent être reliés pour former un groupe dianionique et peuvent former un seul noyau de jusqu'à 30 atomes autres que des atomes d'hydrogène ou un système de noyau multinucléaire de jusqu'à 30 atomes autres que des atomes d'hydrogène ;
L représente O ou S ;
R représente un atome d'hydrogène ou un groupe hydrocarbyle ou hydrocarbyle substitué en C₁ à C₃₀ ;
R¹, R², R³, R⁴, R⁵, R⁶, R⁷ et R⁸ représentent, indépendam-ment, un atome d'hydrogène ou un groupe hydrocarbyle ou hydrocarbyle substitué en C₁ à C₃₀ ;
R⁹ et R¹³ représentent chacun, indépendamment, un atome d'hydrogène ou un groupe hydrocarbyle ou hydrocarbyle substitué en C₁ à C₃₀ ;
R¹⁰, R¹¹, R¹² représentent, indépendamment, un atome d'hydrogène ou un groupe hydrocarbyle ou hydrocarbyle substitué en C₁ à C₃₀ ;
chaque G représente, indépendamment, un atome d'hydrogène, d'halogène ou un groupe hydrocarbyle en C₁ à C₃₀ substitué ou non substitué ;
où deux groupes R adjacents quelconques peuvent former un seul noyau de jusqu'à 8 atomes autres que des atomes d'hydrogène ou un système de noyau multinucléaire de jusqu'à 30 atomes autres que des atomes d'hydrogène.

2. Composé catalyseur selon la revendication 1, dans lequel X et X¹ représentent, indépendamment, un halogène, un alcoolate ou un alkylsulfonate ; L représente O ; R représente un groupe hydrocarbyle en C₁ à C₃₀ ; chaque G représente, indépendamment, un groupe alkyle substitué ou non substitué en C₁ à C₃₀, ou un groupe aryle substitué ou non substitué en C₄ à C₃₀.

3. Composé catalyseur selon la revendication 1 dans lequel le composé catalyseur de métathèse comprend un ou plusieurs des composés suivants : dichlorure de 2-(2,6-diéthylphényl)-3,3,5,5-tétraméthylpyrrolidine-[2-(i-propoxy)-5-(N,N-diméthylaminosulfonyl)phényl]méthy-lène-ruthénium, dichlorure de 2-(mésityl)-3,3,5,5-té-traméthylpyrrolidine-[2-(i-propoxy)-5-(N,N-diméthylami-nosulfonyl)phényl]méthylène-ruthénium, dichlorure de 2-(2-isopropyl)-3,3,5,5-tétraméthylpyrrolidine-[2-(i-pro-poxy)-5-(N,N-diméthylaminosulfonyl)phényl]méthylène-ru-thénium, dichlorure de 2-(2,6-diéthyl-4-fluorophényl)-3,3,5,5-tétraméthylpyrrolidine-[2-(i-propoxy)-5-(N,N-diméthylaminosulfonyl)phényl]méthylène-ruthénium, ou des mélanges de ces composés.

4. Procédé de production d'une alpha-oléfine comprenant la mise en contact d'une huile de graines avec le composé catalyseur selon l'une quelconque des revendications 1 à 3, dans lequel l'alpha-oléfine est de préférence une alpha-oléfine linéaire comportant 4 à 24 atomes de carbone et est de façon plus préférée du butène-1, du décène-1 et ou de l'heptène-1.

5. Procédé selon la revendication 4, dans lequel l'huile de graines est choisie dans le groupe constitué par l'huile de canola, l'huile de maïs, l'huile de soja, l'huile de colza, l'huile d'algue, l'huile d'arachide, l'huile de moutarde, l'huile de tournesol, l'huile de tung, l'huile de périlla, l'huile de pépins de raisin, l'huile de lin, l'huile de carthame, l'huile de cucurbitacées, l'huile de palme, l'huile de graines de Jatropha, l'huile de soja à forte teneur en acide oléique, l'huile de carthame à forte teneur en acide oléique, l'huile de tournesol à forte teneur en acide oléique, des mélanges de graisses et d'huiles animales et végétales, l'huile de ricin, l'huile de ricin déshydratée, l'huile de concombre, l'huile de graines de pavot, l'huile de graines de lin, l'huile de lesque-relle, l'huile de noix, l'huile de graines de coton, l'huile d'écume des prés, l'huile de thon, les huiles de sésame, et leurs mélanges.

6. Procédé selon la revendication 4 comprenant la mise en contact d'un triacylglycéride avec un alcène et le composé catalyseur selon l'une quelconque des revendications 1 à 3, de préférence dans lequel l'alpha-oléfine produite possède au moins un atome de carbone de plus que l'alcène.

7. Procédé selon la revendication 6, dans lequel le triacylglycéride est mis en contact avec un alcool et converti en un ester d'acide gras ou ester alkylique d'acide gras avant la mise en contact avec l'alcène et le composé catalyseur selon l'une quelconque des revendications 1 à 3, ou dans lequel le triacylglycéride est mis en contact avec de l'eau ou un réactif alcalin et converti en un acide gras avant la mise en contact avec l'alcène et le composé catalyseur selon l'une quelconque des revendications 1 à 3.

8. Procédé selon la revendication 4 comprenant la mise en contact d'un acide gras insaturé ou d'un ester d'acide gras insaturé, de préférence un ester alkylique d'acide gras insaturé, tel qu'un ester méthylique d'acide gras, avec un alcène et le composé catalyseur selon l'une quelconque des revendications 1 à 3, de préférence dans lequel l'alpha-oléfine produite possède au moins un atome de carbone de plus que l'alcène.

9. Procédé selon l'une quelconque des revendications 6 à 8, dans lequel l'alcène est de l'éthylène, du propylène, du butène, de l'hexène ou de l'octène.

10. Procédé selon l'une quelconque des revendications 7 à 9, dans lequel le triacylglycéride, l'acide gras, l'ester alkylique d'acide gras, l'ester d'acide gras est dérivé de biodiesel.

11. Procédé selon l'une quelconque des revendications 4 à 10, dans lequel le procédé présente une ou plusieurs des caractéristiques suivantes :
- la productivité du procédé est d'au moins 200 g d'alpha-oléfine linéaire par mmole de catalyseur par heure ;
- la sélectivité du procédé est d'au moins 20 % en poids d'alpha-oléfine linéaire, par rapport au poids du matériau sortant du réacteur ;
- la fréquence d'échange, définie comme étant le nombre de moles d'alpha-oléfine formées par mole de catalyseur, du procédé est d'au moins 10 000 ;
- le rendement, lors de la conversion d'acides gras insaturés, d'esters d'acides gras insaturés, d'esters alkyliques d'acides gras insaturés ou de leurs mélanges, est de 30% ou plus, de préférence de 60% ou plus, ledit rendement étant défini par le nombre de moles d'alpha-oléfine formées par mole d'acides gras insaturés, d'esters d'acides gras insaturés, d'esters alkyliques d'acides gras insaturés ou de leurs mélanges, introduites dans le réacteur ;
- le rendement, lors de la conversion de TAG tels que représentés dans la formule ci-dessous, est de 30% ou plus, ledit rendement étant défini par le nombre de moles d'alpha-oléfine formées divisé par (nombre de moles de R^{a} insaturé + nombre de moles de R^{b} insaturé + nombre de moles de R^{c} insaturé) introduites dans le réacteur, 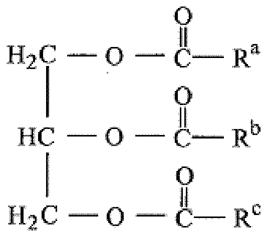 où R^{a}, R^{b} et R^{c} représentent chacun, indépendamment, une chaîne hydrocarbonée saturée ou insaturée.

12. Procédé selon l'une quelconque des revendications 4 à 11 permettant de produire une alpha-oléfine linéaire en C₄ à C₂₄ comprenant la mise en contact d'un matériau d'alimentation avec un alcène choisi dans le groupe constitué par l'éthylène, le propylène, le butène, le pentène, l'hexène, l'heptène, l'octène, le nonène, et leurs mélanges, et d'un composé catalyseur de métathèse selon l'une quelconque des revendications 1 à 3, dans lequel le matériau d'alimentation est un triacylglycéride, un acide gras, un ester alkylique d'acide gras et/ou un ester d'acide gras dérivé d'huile de graines.

13. Procédé selon la revendication 12, dans lequel l'alcène est l'éthylène, l'alpha-oléfine est le 1-butène, le 1-heptène et ou le 1-décène, et le matériau d'alimentation est un ester méthylique d'acide gras, et/ou un ester d'acide gras.
